(19) Europäisches Patentamt
European Patent Office
Office européen des brevets

(11) EP 3 358 513 A1

(12) **EUROPEAN PATENT APPLICATION**
published in accordance with Art. 153(4) EPC

(43) Date of publication:
**08.08.2018 Bulletin 2018/32**

(21) Application number: **16851915.5**

(22) Date of filing: **30.09.2016**

(51) Int Cl.:
***G06Q 10/10*** *(2012.01)* ***A61B 5/00*** *(2006.01)*

(86) International application number:
**PCT/JP2016/079152**

(87) International publication number:
**WO 2017/057746 (06.04.2017 Gazette 2017/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.09.2015 JP 2015194368**
**05.08.2016 JP 2016154333**

(71) Applicant: **Nittsu System Co., Ltd.**
**Nagoya-shi, Aichi 460-0008 (JP)**

(72) Inventors:
- **KAMURA, Minoru**
  **Nagoya-shi**
  **Aichi 460-0008 (JP)**
- **KAMURA, Kenshi**
  **Nagoya-shi**
  **Aichi 460-0008 (JP)**
- **ISHITANI, Shingo**
  **Nagoya-shi**
  **Aichi 460-0008 (JP)**

(74) Representative: **Groth & Co. KB**
**P.O. Box 6107**
**102 32 Stockholm (SE)**

# (54) LABOR MANAGEMENT SYSTEM, LABOR MANAGEMENT METHOD, AND LABOR MANAGEMENT METHOD

(57) A labor management system wherein a first group of items includes a group of attendance items and/or a group of personnel items, and a second group of items includes a group of physical checkup items and/or a group of stress check items. First data indicates the result for each first item constituting the first group of items. The first data indicates the result for each first item for an employee as a history. Second data indicates the result for each second item constituting the second group of items. The second data indicates the result for each second item for the employee. An input unit receives, as inputs, at least one first item and at least one second item, as specification items. An output control unit controls outputs to an output unit such that the output unit outputs a graph showing the results for each specification item. The graph includes the first data and the second data. The position of the result for each specification item on a time axis is aligned with the position of the results for the other specification items.

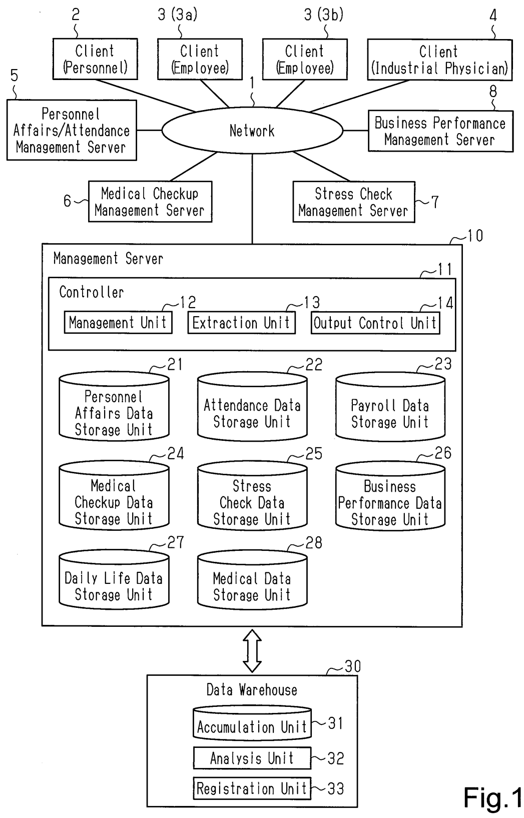


Fig.1

EP 3 358 513 A1

## Description

TECHNICAL FIELD

The present invention relates to a labor management system, a labor management method, and a labor management program for managing the health of employees by referring to data on an attendance state, medical checkup results, stress check results, and the like.

BACKGROUND ART

Attendance management of employees has been conducted in each company. In attendance management, a computer system is used to manage total working hours, overtime hours, the number of times of holiday work, the number of times of late arrival, the number of times of early leaving, and the like. Also, companies regularly provide medical checkups for the purpose of managing the health of employees. Further, a stress check is conducted for employees who may be under a significant amount of stress in the course of their work. Additionally, if necessary, face-to-face guidance is conducted with an industrial physician or the like. As described above, currently, companies are requested to provide employees with medical checkups and stress checks in addition to attendance management to perform labor management of employees.

In this respect, patent document 1 describes a health management system used, for example, in a company for employees. In the health management system of patent document 1, medical checkup results of employees and an attendance state of employees are managed to evaluate the fatigue state of the employees by referring to these data. In a case where measures need to be taken for an employee, in addition to the employee, the supervisor of the employee is also requested to take measures.

PRIOR ART DOCUMENT

Patent Document

Patent Document 1: Japanese Laid-Open Patent Publication No. 2003-256578

SUMMARY OF THE INVENTION

Problems that are to be Solved by the Invention

However, in the health management system of patent document 1, which evaluates the fatigue state from the medical checkup results and the attendance state, users of the system cannot easily understand the relationship between the medical checkup results and/or stress check results with the attendance state and/or personnel affairs. The relationship indicated by these is an important factor in improving the health condition and/or working state of employees in the future. Therefore, the above-described labor management system is strongly desired to assist in understanding of this relationship.

An object of the present invention is to provide a labor management system, a labor management method, and a labor management program capable of assisting in understanding of the relationship between the medical checkup results or stress check results and the attendance state or personnel affairs.

Means for Solving the Problem

To achieve the above object, a first aspect of the present invention is a labor management system including a first item group, a second item group, first data, second data, a storage unit, an input unit, and an output control unit. The first item group includes at least one of an attendance item group and a personnel affairs item group. The second item group includes at least one of a medical checkup item group and a stress check item group. The first data shows a result of each of first items that configure the first item group. The first data is data that shows the result of each of first items of an employee as a history. The second data shows a result of each of second items that configure the second item group. The second data is data that shows the result of each of second items of the employee as a history. The storage unit stores the first data and the second data. In the input unit, at least one of the first items and at least one of the second items are respectively input as designated items. The output control unit controls output to an output unit so that the output unit outputs a graph showing a result of each of the designated items. The graph includes the first data and the second data stored in the storage unit. The result of each of the designated items is positioned on a time axis so that the designated items are in conformance.

A second aspect of the present invention is a labor management method that includes specifying at least one of an attendance item group and a personnel affairs item group as a first item group. The method further includes specifying at least one of a medical checkup item group and a stress check item group as a second item group. The method further includes specifying first data showing a result of each of first items that configure the first item group. The first data is data showing the result of each of the first items of an employee as a history. The method further includes specifying second data showing a result of each of second items that configure the second item group. The second data is data showing the result of each of the second items of the employee as a history. The method further includes storing the first data and the second data in a storage unit. The method further includes inputting at least one of the first items and at least one of the second items respectively as designated items with an input unit. The method further includes controlling an output to an output unit with an output control unit so that the output unit outputs a graph showing a result of each of the designated items. The graph includes the first data and the second data stored in the storage unit. The result of each of the designated items is positioned on a time axis so that the designated items are in conformance.

A third aspect of the present invention is a labor management program that includes specifying at least one of an attendance item group and a personnel affairs item group as a first item group. The program further includes specifying at least one of a medical checkup item group and a stress check item group as a second item group. The program further includes specifying first data showing a result of each of first items that configure the first item group. The first data is data showing the result of each of the first items of an employee as a history. The program further includes specifying second data showing a result of each of second items that configure the second item group. The second data is data showing the result of each of the second items of the employee as a history. The program further includes having a computer function as a means for storing the first data and the second data in a storage unit. The program further includes inputting at least one of the first items and at least one of the second items respectively as designated items with an input unit. The program further includes having a computer function as a means for having an output control unit control an output unit so that the output unit outputs a graph showing a result of each of the designated items. The graph includes the first data and the second data stored in the storage unit. The result of each of the designated items is positioned on a time axis so that the designated items are in conformance.

Further, the labor management method according to the present invention is carried out by the above-described labor management system.

Furthermore, the labor management program according to the present invention is used in the above-described labor management system. The labor management program is widely distributed via a network and/or removable storage media such as optical disks to be installed on and run by a computer such as a server.

BRIEF DESCRIPTION OF THE DRAWINGS

Fig. 1 is a block diagram showing an overview of a system according to an embodiment of the present invention.

Fig. 2A shows a configuration of a personnel affairs data storage unit of Fig. 1, Fig 2B shows a configuration of an attendance data storage unit of Fig. 1, and Fig. 2C shows a configuration of a payroll data storage unit of Fig. 1.

Fig. 3A shows a configuration of a medical checkup data storage unit of Fig. 1, Fig 3B shows a configuration of a stress check data storage unit of Fig. 1, Fig. 3C shows a configuration of a business performance data storage unit of Fig. 1, Fig. 3D shows a configuration of a daily life data storage unit of Fig. 1, and Fig. 3E shows a configuration of a medical data storage unit of Fig. 1.

Fig. 4 is a flowchart showing processing for storing data in each of the storage units shown in Fig. 2 and Fig 3.

Fig. 5 is a flowchart showing processing in a case where the system is used directly by an employee and where an abnormality is found in a medical checkup.

Fig. 6 is a diagram showing a state that a report is displayed on a monitor in a first output mode in which graphs of selected item data are aligned vertically.

Fig. 7 is a diagram showing a state that a report is displayed on a monitor in a second output mode in which graphs of selected item data are superimposed.

Fig. 8 is a flowchart showing processing in a case where the system is used directly by an employee and where an abnormality is found in a stress check.

Fig. 9 is a flowchart showing processing in a case where the system is used directly by an employee and where the health condition is checked regularly.

Fig. 10 is a flowchart showing processing in a case where the system is used directly by an employee and where a subjective symptom such as a poor physical condition is present.

Fig. 11 is a flowchart showing processing in a case where the system is used by a person in charge of personnel affairs and where overwork and/or poor work performance are detected.

Fig. 12 is a flowchart showing processing in a case where the system is used by a person in charge of personnel affairs and where an employee returns from a leave of absence.

Fig. 13 is a flowchart showing processing in a case where the system is used by a person in charge of personnel affairs and where performance improvement measures are considered.

Fig. 14 is a flowchart showing processing in a case where the system is used by an industrial physician when conducting consultation, for example, about a poor physical condition.

Fig. 15 is a diagram showing a state that a report is displayed on a monitor in a first output mode in which enhancement processing is applied to an item showing an abnormality.

Fig. 16 is a diagram showing a modified example of the first output mode in which graphs of selected item data are aligned vertically and icons are used (refer to Fig. 6).

Fig. 17 is a diagram showing a modified example of the second output mode in which graphs of selected item data are superimposed and icons are used (refer to Fig. 7).

Fig. 18 is a diagram showing a modified example of an output mode in which graphs of items are superimposed for each large category.

Fig. 19 is a diagram showing an output mode in which "Total overtime hours" is changed from a yearly basis to a monthly basis.

Fig. 20A is a modified example of the output mode in which graphs of selected item data are superimposed and icons are used, and Fig 20B is a diagram showing a list of icons used in Fig. 20A.

Fig. 21 is a flowchart showing procedures of statistical analysis processing 1.

Fig. 22 is a flowchart showing procedures in a case where medical checkup results are viewed by an employee.

Fig. 23 is a diagram showing an output mode of a report.

Fig. 24 is a flowchart showing procedures of statistical analysis processing 2.

Fig. 25 is a diagram showing a multiple regression analysis.

Fig. 26 is a flowchart of processing for projecting the future of an individual employee.

EMBODIMENTS OF THE INVENTION

Hereinafter, a description will be given of one embodiment in which a labor management system, a labor management method, and a labor management program are embodied with reference to Fig. 1 to Fig. 26. In the present embodiment, a description will be given of a labor management system in which information related to personnel affairs, information related to attendance, information related to medical checkup results, and/or information related to stress check results of employees are used to manage the health of employees in a comprehensive manner, that is, to manage the health of employees from both physical and mental aspects.

As shown in Fig. 1, a management server 10 used in the labor management system is connected via a network 1 to a client terminal 2 used, for example, by a person in charge of personnel affairs, client terminals 3a, 3b (hereinafter, also collectively referred to as a client terminal 3) used, for example, by employees, and a client terminal 4 used, for example, by an industrial physician. The client terminals 2, 3, 4 each include an output unit and an input unit. The output unit is a means for outputting various types of information and configured, for example, by a display and a printer. The input unit is a means for inputting various types of information and configured, for example, by a keyboard, a pointing device, and a communication interface. Also, the client terminal 3 and servers 5 to 8, which input various types of data to the management server 10, serve as the input unit.

The client terminal 2 for a person in charge of personnel affairs, the client terminal 4 for an industrial physician, and the client terminal 3a for an employee are each, for example, a desktop or laptop computer terminal capable of displaying on a monitor a report containing the medical checkup results of employees or printing the report by using a printer for checking.

The client terminal 3b for an employee is a small-sized information processing terminal such as a smartphone, a mobile phone, or a laptop computer carried by an employee, or an eyeglass-type or wristwatch-type wearable terminal. The client terminal 3b includes a sensor for measuring physical data such as the number of walking steps, amount of exercise, blood pressure, and pulse rate and stores the detected values, for example, in an internal memory. The client terminal 3b also stores activity data such as hours of sleep, sleep time, and wake-up time as well as dietary data such as the number of meals per day, meal time, and intake calories. Further, habit data such as alcohol intake amounts and smoking amounts is also stored. Furthermore, medical data such as time of having a medical checkup and/or a medical examination, time of starting medication, time of onset of disease, and time of cure and/or time of interview with a physician is also stored. The client terminal 3b stores such daily life data and/or the medical data on employees and sends the daily life data and/or the medical data regularly or in accordance with an operation performed by an employee to the management server 10 via the client terminal 3a of the employee or directly.

The management server 10 is also connected via the network 1 to a personnel affairs/attendance management server 5, a medical checkup management server 6, a stress check management server 7, and a business performance management server 8, which manages business performance of the company in its entirety and/or employees. An employee code is uniquely given to each employee, and each of the servers 5 to 8 performs management in association with the employee codes.

The personnel affairs/attendance management server 5 accumulates and manages data on personnel affairs and/or attendance of employees. Specifically, the personnel affairs/attendance management server 5 manages, for each employee, item data related to company events such as date of joining the company, date of transfer to a different department, and promotion date and also manages item data related to private events such as date of marriage, date of divorce, date of child birth, date of start of nursing, and date of end of nursing. Further, the personnel affairs/attendance management server 5 manages hours data such as total working hours and total overtime hours, day/frequency count data such as the number of days worked and the number of times of business trips, and time data such as clock-in time and clock-out time, as attendance data for each employee. Furthermore, the personnel affairs/attendance management server 5 manages payroll data for each employee. The personnel affairs/attendance management server 5 sends the management server 10 personnel affairs data such as the data related to company events and data related to private events, the day/frequency count data, the attendance data such as clock-in time and clock-out time, and/or the payroll data regularly or in accordance with an operation performed by an operator.

The medical checkup management server 6 manages, for each employee, medical checkup data, which are the results of medical checkups regularly performed on employees. Specifically, the medical checkup management server 6 manages item data, which are the results of items such as body weight, height, BMI, and uric acid values that configure the medical checkup data. The medical checkup management server 6 sends the medical checkup data to the management server 10 regularly or in accordance with an operation performed by an operator.

The stress check management server 7 manages, for each employee, stress check data, which are stress check results of employees. Specifically, the stress check management server 7 manages item data, which are the results of items such as results of work load determination, results of work resource (work level) determination, results of work resource (department) determination, results of work resource (office) determination, and results of comprehensive determination that configure the stress check data. The work resources indicate factors in the organization such as a decrease in work load, mitigation of adverse work load, and enhancement of motivation. Specifically, question items such as "Is there support available from your supervisor?," "Is there support available from your colleagues?," and "Are individuals respected?" are evaluated and rated as scores (for example, one point to four points). The stress check management server 7 performs stress checks on all employees regularly or, for example, employees who worked in excess of specified overtime hours, and accumulates and manages response data for the stress checks sent from the client terminal 3 for the employee. Then, the stress check management server 7 sends the stress check data to the management server 10 regularly or in accordance with an operation performed by an operator.

The business performance management server 8 manages item data on, for example, sales amount, sales volume, and ordered amount, as business performance data of a company in its entirety, each office, each department, and each employee. The business performance management server 8 sends the business performance data to the management server 10 regularly or in accordance with an operation performed by an operator.

The management server 10 to which the above-described devices are connected via the network 1 is an ordinary server, a computer system configured by hardware such as a CPU, ROM, RAM, and hard disks, and a computer system for performing management of information related to human resources of the company. The management server 10 includes a controller 11, a personnel affairs data storage unit 21, an attendance data storage unit 22, a payroll data storage unit 23, a medical checkup data storage unit 24, a stress check data storage unit 25, a business performance data storage unit 26, a daily life data storage unit 27, and a medical data storage unit 28.

The controller 11 includes a management unit 12, an extraction unit 13, and an output control unit 14. The controller 11 may be realized, for example, by a circuitry, that is, one or more dedicated hardware circuitries such as ASIC, one or more processing circuitries operating in accordance with a computer program (software), or a combination of both. The processing circuitry has a CPU and a memory (ROM, RAM, etc.) that stores programs executed by the CPU. The memory, that is, a computer-readable medium, includes any available media that is accessible by general-purpose or dedicated computers.

The management unit 12 regularly sends a request for sending data to the personnel affairs/attendance management server 5, the medical checkup management server 6, the stress check management server 7, and the business performance management server 8 and stores and manages data sent from each of the servers in the respective storage units 21 to 26. The management unit 12 also stores daily life data sent from the client terminal 3a and/or the client terminal 3b of employees in the daily life data storage unit 27.

The extraction unit 13 extracts an employee in which an abnormality is detected in a medical checkup and/or a stress check. The extraction unit 13 performs the extraction processing, for example, regularly or in accordance with an operation performed by an operator. It also extracts the data of the corresponding employee from the personnel affairs data storage unit 21, the attendance data storage unit 22, the payroll data storage unit 23, the medical checkup data storage unit 24, the stress check data storage unit 25, the business performance data storage unit 26, the daily life data storage unit 27, and the medical data storage unit 28.

The output control unit 14 generates a report for each employee as output data based on information related to personnel affairs, information related to attendance, information related to medical checkup results, and/or information related to stress check results. The report includes visible data such as text data, image data, and/or video data, and is displayed on monitors of the client terminals 2, 3, 4 or output on a sheet of printing paper by using a printer connected to the client terminals 2, 3, 4 or connected to the network 1. Further, the output data is output to a portable storage medium such as an optical disk, a USB memory, or a memory card.

The management server 10 is further connected to a data warehouse 30 via a network. Data stored in each of the storage units 21 to 26 is sent to the data warehouse 30 from the management server 10 regularly or in accordance with an operation performed by an operator. The data warehouse 30 organizes and accumulates the received data, which has been stored in each of the storage units 21 to 26.

The data warehouse 30 is an ordinary server and is a computer system configured by hardware such as a CPU, ROM, RAM, and a large-capacity hard disk, and accumulates data sent from the management server 10 in a systematic manner to analyze the data. The data warehouse 30 stores the data sent from the management server 10 in an accumulation unit 31, which is, for example, a large-capacity hard disk. The data warehouse 30 includes an analysis unit 32 that performs a statistical analysis on data accumulated in the accumulation unit 31. Specifically, the analysis unit 32 calculates a correlation of, for example, each item of the personnel affairs data and/or each item of the attendance data with respect to each medical checkup item of a medical checkup and/or each stress check item of a stress check. Then, it extracts each item of the personnel affairs data and/or each item of the attendance data that are highly correlated with each medical checkup item of the medical checkup and/or each check item of the stress check as a related item. The data warehouse 30 includes a registration unit 33 that registers a combination of highly-correlated items.

As shown in Fig. 2A, the personnel affairs data storage unit 21 stores a personnel affairs record 21a that records personnel affairs data, or an attribute of each employee. The personnel affairs record 21a is recorded in a case where personal information related to an employee is registered. The personnel affairs record 21a stores various types of personnel affairs item data, which is the history of personnel affairs, in association with the employee code. Specifically, the personnel affairs record 21a records, for example, a name, affiliation, a job title, a working form, and contact information, and also records, for example, company events that occur for each employee within the company and private events that occur privately for each employee. The company events are data of time and date containing, for example, date of joining a company, date of transferring to a different department, and date of promotion. The private events are data of time and date containing, for example, date of marriage, date of divorce, and date of child birth. Each item that is stored in the personnel affairs data storage unit 21 is a first item, an aggregation of the first items is a first item group, and item data showing results of the first items as a history is first data.

As shown in Fig. 2B, the attendance data storage unit 22 stores an attendance record 22a in which attendance data on attendance of each employee is recorded. The attendance record 22a is in association with the employee code, and item data as a history of attendance is recorded in the attendance record 22a. Specifically, item data on, for example, total working hours, total overtime hours, late-night overtime hours, and holiday working hours is recorded as hours data. Also, item data on, for example, the number of days worked, the number of days of out-of-office work, the number of days of business trips, the number of times of holiday work, and the number of times of night shift is recorded as day/frequency count data. Further, item data, for example, on clock-in time, clock-out time and paid leave use rate is recorded as time data. Each item that is stored in the attendance data storage unit 22 is a first item, an aggregation of the first items is a first item group, and item data showing results of the first items as a history is first data.

As shown in Fig. 2C, the payroll data storage unit 23 stores a payroll record 23a that records payroll data on each employee. Data is recorded as a history of salary in the payroll record 23a in association with the employee code. Specifically, the payroll data storage unit 23 stores item data of the payroll data on, for example, monthly salary, bonus, and annual salary of each employee. Each item that is stored in the attendance data storage unit 22 is a first item, an aggregation of the first items is a first item group, and item data showing results of the first items as a history is first data.

As shown in Fig. 3A, the medical checkup data storage unit 24 stores a medical checkup record 24a that records the medical checkup data, which are the results of a medical checkup of each employee. Data showing a history of results of the medical checkup is recorded in the medical checkup record 24a in association with the employee code. Specifically, the medical checkup item data separated by items such as body weight, height, abdominal circumference, and BMI is recorded. Further, the medical checkup data storage unit 24 stores image data of X-ray examinations such as a chest X-ray and a stomach X-ray, image data of MRI examinations such as brain, spinal cord, limb as well as pelvic cavity, for example, a uterus, ovaries, and prostate, and video data of ultrasound scanning, for example, liver, gallbladder, kidneys, pancreas, urinary bladder, prostate, a uterus, and ovaries. In addition, image data of CT scanning may also be recorded. The medical checkup record 24a may also record medical examination results containing such images and/or videos. Each item that is stored in the medical checkup data storage unit 24 is a second item, an aggregation of the second items is a second item group, and item data showing results of the second items as a history is second data.

As shown in Fig. 3B, the stress check data storage unit 25 stores a stress check record 25a that records stress check data, which are the results of a stress check of each employee. Data on a history of the stress check is recorded in the stress check record 25a in association with the employee code. Specifically, the check item data separated by items such as comprehensive determination, work load determination, and work resource determination (work level, department, office) is recorded. Each item that is stored in the stress check data storage unit 25 is a second item, an aggregation of the second items is a second item group, and item data showing results of the second items as a history is second data.

As shown in Fig. 3C, the business performance data storage unit 26 stores a business performance record 26a that records item data on, for example, sales amount, sales volume, and ordered amount, as business performance data of a company in its entirety, each office, each department, and each employee. In a case of business performance of an employee, data on a history of business performance is recorded in the business performance record 26a in association with the employee code. Specifically, the business performance record 26a stores item data on, for example, sales amount, sales volume, an ordered amount, ordered volume, and productivity. Each item that is stored in the business performance data storage unit 26 is a first item, an aggregation of the first items is a first item group, and item data showing results of the first items as a history is first data.

As shown in Fig. 3D, the daily life data storage unit 27 stores a daily life record 27a that records daily life data of each employee. The daily life record 27a stores item data such as activity data, physical data, dietary data, and habit data in association with the employee code. The activity data includes, for example, the number of walking steps, amount of exercise, and consumed calories. The physical data includes, for example, body weight, body fat percentage, and blood pressure. The dietary data includes meal time, the number of meals per day, and intake calories. The habit data includes alcohol intake amounts and smoking amounts. Thus, for example, with regard to blood pressure and/or body weight, daily blood pressure and/or body weight is manageable in addition to the blood pressure and/or body weight at the time of a medical checkup. Each item that is stored in the daily life data storage unit 27 is a second item, an aggregation of the second items is a second item group, and item data showing results of the second items as a history is second data.

As shown in Fig. 3E, the medical data storage unit 28 stores a medical record 28a that records medical data on each employee. The medical record 28a stores item data on, for example, time of having a medical checkup and/or a medical examination, time of starting medication, time of onset of disease, time of cure, and/or time of interview with a physician as medical events in association with the employee code. Each item that is stored in the medical data storage unit 28 is a second item, an aggregation of the second items is a second item group, and item data showing results of the second items as a history is second data.

As described so far, item data of personnel affairs/attendance/business performance-related data such as the personnel affairs data, the attendance data, and the business performance data are classified as the first data. And, each item data of health-related data such as the medical checkup data, the stress check data, the daily life data, and the medical data are classified as the second data. The daily life data and the medical data may be treated as the first data.

Next, a description will be given of operations when the above-described system labor management is carried out.

Data Management Processing

A method for storing various types of data in each of the storage units 21 to 27 of the labor management system will be described with reference to Fig. 4.

In step S1, the management unit 12 of the management server 10 receives item data of the personnel affairs data sent from the personnel affairs/attendance management server 5 and stores the data in the personnel affairs data storage unit 21 in association with the employee code. In step S2, the management unit 12 receives item data of the attendance data sent from the personnel affairs/attendance management server 5 and stores the data in the attendance data storage unit 22 in association with the employee code. In step S3, the management unit 12 receives item data of the payroll data sent from the personnel affairs/attendance management server 5 and stores the data in the payroll data storage unit 23 in association with the employee code.

In step S4, the management unit 12 of the management server 10 receives medical checkup item data of the medical checkup data sent from the medical checkup management server 6 and stores the data in the medical checkup data storage unit 24 in association with the employee code. In step S5, the management unit 12 receives check item data of the stress check data sent from the stress check management server 7 and stores the data in the stress check data storage unit 25 in association with the employee code. In step S6, the management unit 12 receives item data of the daily life data sent from the client terminal 3 for an employee and stores the data in the daily life data storage unit 27 in association with the employee code. In step S7, the management unit 12 receives item data of the medical data sent from the client terminal 3 for an employee and stores the data in the medical data storage unit 28 in association with the employee code. For example, the data on time of having a medical checkup and/or a medical examination, time of starting medication, time of onset of disease, and time of cure and/or time of interview with a physician may be obtained from the medical checkup management server 6 in addition to the client terminal 3 for an employee. In step S8, the management unit 12 receives item data of the business performance data sent from the business performance management server 8 and stores the data, for example in a case of business performance of an employee, in the business performance data storage unit 26 in association with the employee code. In step S9, the management unit 12 sends various types of data stored in each of the storage units 21 to 27 to the data warehouse 30 regularly or in accordance with an operation performed by an operator. The data warehouse 30 stores the received data in the accumulation unit 31. The analysis unit 32 analyzes the data accumulated in the accumulation unit 31. The data analysis will be described in detail later.

Case where Employee Directly Uses System

Case where Abnormality is found in Medical Checkup

With reference to Fig. 5, a description will be given of a case where the system is used directly by an employee and where an abnormality is found in a medical checkup.

When an employee operates the client terminal 3 and sends, for example, the employee code to the management server 10, the extraction unit 13 accesses the medical checkup data storage unit 24 to search for the medical checkup record 24a of the received employee code. In step S11, the extraction unit 13 judges whether or not an abnormal value is found in the individual medical checkup items data recorded in the medical checkup record 24a. Specifically, some of the medical checkup items, for example, body fat percentage, uric acid value, LDL cholesterol, and HLD cholesterol, each have a proper range of values. If an item is out of the proper range, a caution is provided to the employee. The medical checkup data storage unit 24 records the proper range of medical checkup item data in a specification record. The extraction unit 13 judges whether or not the medical checkup item data is abnormal with reference to the specification record.

In step S12, the extraction unit 13 extracts medical checkup item data in which an abnormality is found and, in step S13, extracts medical checkup item data related to the medical checkup item data found to have an abnormality. For example, when an abnormal body fat percentage is found, for example, LDL cholesterol, HLD cholesterol, and total cholesterol are extracted as the related medical checkup item data. In the medical checkup data storage unit 24, predetermined medical checkup item data is in association with related medical checkup items. The extraction unit 13 extracts medical checkup item data related to a medical checkup item found to have an abnormality with reference to the above association. When no abnormality is found in the medical checkup item data, in step S14, the extraction unit 13 extracts a general and predetermined medical checkup item that has been specified in advance.

In step S15, the extraction unit 13 accesses the personnel affairs data storage unit 21, searches for the personnel affairs record 21a of the received employee code, and extracts the personnel affairs item data of the personnel affairs record 21a. In step S16, the extraction unit 13 accesses the attendance data storage unit 22, searches for the attendance record 22a of the received employee code, and extracts the attendance item data of the attendance record 22a. In step S17, the extraction unit 13 accesses the daily life data storage unit 27, searches for the daily life record 27a of the received employee code, and extracts each item data of the daily life record 27a. In step S15 to step S17, the extraction unit 13 extracts the predetermined item data that has been set in advance from each record and further extracts items selected by the user. All of the items extracted may be freely selected by the user. In addition, the predetermined item data that has been set in advance may be an item extracted, for example, by statistical analysis processing, which will be described later.

In step S18, the output control unit 14 of the management server 10 generates a report as output data that is sent to the client terminal 3 requesting the report. Specifically, the output control unit 14 generates a report by selecting one of output modes, that is, a first output mode in which graphs of selected item data are aligned vertically and a second output mode in which graphs of selected item data are superimposed. The output control unit 14 sends the report to the client terminal 3 in the requested output mode. At the client terminal 3, the employee may read the report by displaying the report on a monitor or printing the report by using a printer.

In a case where an abnormality is found in a medical checkup, for example, findings of a physician may be described. Such findings are also stored in the medical checkup data storage unit 24 in association with the employee code. In step S11, instead of extracting a medical checkup item found to have an abnormality or a medical checkup item related to the abnormal medical checkup item, the extraction unit 13 may extract a medical checkup item to which findings are given and a medical checkup item related to the medical checkup item to which findings are given. Further, when an abnormality is found, for example, in a chest X-ray, a stomach X-ray, MRI examinations of the brain, and/or ultrasound scanning of the liver, the extraction unit 13 may also extract the corresponding item and a medical checkup item related to the corresponding item.

First Output Mode

Fig. 6 is a diagram showing a state that a report is displayed on a monitor in the first output mode, in which graphs of selected item data are aligned vertically. A report 41 includes a radio button 42. The radio button 42 may be used to select one of output modes, that is, the first output mode of a vertically-arranged display and a second output mode of a superimposed display. Here, the first output mode is selected to provide a vertically-arranged display. Further, in the report 41, a pull-down menu may be used to select a combination pattern. A first pull-down menu 43 allows for a selection of, for example, "Check stress check results" in addition to "Check medical checkup results." Here, "Check medical checkup results" is selected. Also, a second pull-down menu 44 is provided to select target data to be displayed. Any one of "Attendance," "Personnel affairs," "Stress check results," "Medical checkup," and "Daily life" may be selected from the second pull-down menu 44 as a designated item of large categories. Then, with regard to the selected large category, item data may be further selected as a subcategory of the designated item. In the example shown in Fig. 6, "Attendance," "Personnel affairs," and "Medical checkup" are selected. The output control unit 14 accesses the attendance data storage unit 22, the personnel affairs data storage unit 21, and the medical checkup data storage unit 24 to extract data of the selected subcategory using the extraction unit 13 and adds the data to the report 41. Specifically, "Body weight," "Total cholesterol," and "LDL cholesterol" are selected in the "Medical checkup." The output control unit 14 extracts item data of "Body weight," "Total cholesterol," and "LDL cholesterol" using the extraction unit 13 and adds the data to the report 41. Further, "Total overtime hours" is selected as a subcategory in "Attendance." The output control unit 14 extracts item data of "Total overtime hours" using the extraction unit 13 and adds the data to the report 41.

A related item such as "Total overtime hours" displayed as the item related to "Medical checkup" may be an item selected, for example, by an employee or may be an item extracted by statistical analysis processing described later.

In the first output mode of a vertically-arranged display, a graph of "Body weight," a graph of "Total cholesterol," a graph of "LDL cholesterol," and a graph of "Total overtime hours" are generated one by one sequentially from the above, and items of "Transfer to different department," "Promotion," and "Change of residence" are also aligned vertically. Further, data for a period of time from the year 2009 to the year 2014 is extracted so as to be consistent in a range of a time axis, and for this period of time, the positions of the horizontal axes are aligned in conformance on the time axis. In the above-described report 41, the horizontal axis is the time axis. With the position of each item data aligned in conformance on the time axis, the graphs are aligned in a direction orthogonal to the time axis. Thus, for example, when a change is found in the medical checkup results, other situations are easily understood. In the example shown in Fig. 6, the body weight decreased in the year 2012. It is instantly understood that the employee was promoted to a manager in that year, and further, that the total overtime hours increased in that year.

Further, an upper limit line 45 of the proper range is added as an index to the graph of "Total cholesterol" and the graph of "LDL cholesterol." This facilitates understanding of the graph of "Total cholesterol" and the upper limit value of "LDL cholesterol." A subcategory that is highly related to the medical checkup items such as "Total overtime hours" is automatically displayed by statistical analysis. This prevents overlooking of a subcategory in a large category different from the "Medical checkup" such as "Attendance," which is related to the worsening of the medical checkup results.

Second Output Mode

Fig. 7 is a diagram showing a state that a report is displayed on a monitor in the second output mode in which graphs of selected item data are superimposed. In a report 51, the radio button 42 is used to select the second output mode, which provides a superimposed display. Items similar to those selected in Fig. 6 are also selected in Fig. 7. The report 51 includes a radio button 52, by which a scale on the vertical axis is selected. Here, "Total cholesterol" is selected, and the scale of the vertical axis is set so as to correspond to "Total cholesterol." In the example shown in Fig. 7, the position of the graph of each item is aligned in conformance on the time axis, and further, the graphs are superimposed. Thus, it is instantly understood that, although the body weight decreased in the year 2012, the employee was promoted to a manager in that year, and further, that total overtime hours increased in that year.

In the first output mode shown in Fig. 6 and the second output mode shown in Fig. 7, "Stress check results" and/or "Daily life item" may be additionally selected as a large category. Also, when "Stress check results" is selected, an item such as "Work load determination" may also be selected as a subcategory. Further, when "Daily life item" is selected, items such as "The number of walking steps" and "Amount of exercise" may be selected as subcategories. Thus, the relationship between medical checkup results with other items is further understood from the reports 41 and 51. Further, in a state that one of the first output mode shown in Fig. 6 and the second output mode shown in Fig. 7 is displayed on the monitor of the client terminal 3, when the other output mode, which is not displayed with the radio button 42, is selected by using an operation portion such as a mouse, the output control unit 14 generates output data in the output mode that is newly selected. Then, the client terminal 3 displays a report on the monitor.

Case where Abnormality is found in Stress Check

With reference to Fig. 8, a description will be given of a case where the labor management system is used directly by an employee and where an abnormality is found in a stress check.

When an employee operates the client terminal 3 and sends, for example, the employee code to the management server 10, the extraction unit 13 accesses the stress check data storage unit 25 to search for the stress check record 25a of the received employee code. In step S21, the extraction unit 13 judges whether or not an abnormal value is found in each check item data recorded in the stress check record 25a. Specifically, some of the check item data each have a proper range of values. If check item data is out of the proper range, a caution is provided to the employee. The stress check data storage unit 25 specifies the proper range for each check item data in a specification record, and, the extraction unit 13 judges whether or not check item data is abnormal with reference to the specification record.

In step S22, the extraction unit 13 extracts check item data in which an abnormality is found. When no abnormality is found in the check item data, in step S23, it extracts general and predetermined check item data that has been specified in advance. In step S24, the extraction unit 13 extracts main medical checkup item data. In the stress check data storage unit 25, predetermined check item data is in association with related medical checkup item. The extraction unit 13 extracts the medical checkup item data related to the check item data found to have an abnormality with reference to the association. The related medical checkup item may be an item extracted, for example, by statistical analysis processing, which will be described later.

In step S25, the extraction unit 13 accesses the personnel affairs data storage unit 21 to search for the personnel affairs record 21a of the received employee code and extracts personnel affairs item data of the personnel affairs record 21a. This is because, for example, personnel reshuffle and job title may affect stress. In step S26, the extraction unit 13 accesses the attendance data storage unit 22 to search for the attendance record 22a of the received employee code and extracts attendance item data of the attendance record 22a. This is because, for example, total overtime hours per month may continue to exceed predetermined hours set in advance. In step S27, the extraction unit 13 accesses the payroll data storage unit 23 to search for the payroll record 23a of the received employee code and extracts item data of the payroll record 23a. In step S28, the extraction unit 13 accesses the business performance data storage unit 26 and extracts business performance item data recorded in the business performance record 26a of the employee. For example, sales amount, sales volume, ordered amount, and ordered volume of the employee in each month or each year are extracted. In step S29, the extraction unit 13 accesses the daily life data storage unit 27 to search for the daily life record 27a of the received employee code and extracts item data of the daily life record 27a. In step S25 to step S29, the extraction unit 13 extracts predetermined item data that has been set in advance from each record and further extracts items selected by the user. All of the items extracted may be freely selected by the user. In addition, the predetermined item data that has been set in advance may be an item extracted, for example, by statistical analysis processing, which will be described later.

In step S30, the output control unit 14 of the management server 10 generates a report in the first output mode or a report in the second output mode as output data that is sent to the client terminal 3 requesting the report and sends the report to the client terminal 3. The client terminal 3 displays the report on a monitor or prints the report by using a printer to allow the employee to read the report. This allows the employee to determine what affects stress and how the stress affects the physical aspects.

Case where Health Condition is Regularly Checked

With reference to Fig. 9, a description will be given of a case where the labor management system is used directly by an employee to regularly check health condition.

In step S31, the extraction unit 13 judges whether or not the current time and date is the checking time and date of medical checkup results desired by each employee. For example, data on the checking time and date is recorded in the medical checkup record 24a, and upon arrival of the checking time and date, in step S32, the extraction unit 13 accesses the medical checkup data storage unit 24 and extracts main medical checkup items recorded in the medical checkup record 24a of the received employee code. In step S33, the extraction unit 13 accesses the stress check data storage unit 25 and extracts main check item data recorded in the stress check record 25a of the received employee code. In step S34, the extraction unit 13 accesses the daily life data storage unit 27 and extracts item data of the daily life record 27a of the received employee code. In step S32 to step S34, the extraction unit 13 extracts predetermined item data that has been set in advance from each record and further extracts items selected by the user. All of the items extracted may be freely selected by the user. In addition, the predetermined item data that has been set in advance may be an item extracted, for example, by statistical analysis processing, which will be described later.

In step S35, the output control unit 14 of the management server 10 generates a report in the first output mode or a report in the second output mode as output data that is sent to the client terminal 3 requesting the report and sends the report to the client terminal 3. The client terminal 3 displays the report on a monitor or prints the report by using a printer to allow the employee to read the report. This allows the employee to determine regularly whether or not there is any change in the physical and/or stress aspects.

Case where Subjective Symptom Such as Poor Physical Condition is Found

With reference to Fig. 10, a description will be given of a case where the labor management system is used directly by an employee and where a subjective symptom such as a poor physical condition is found.

When an employee operates the client terminal 3 and sends, for example, the employee code to the management server 10, in step S41, the extraction unit 13 accesses the medical checkup data storage unit 24 and extracts main medical checkup items recorded in the medical checkup record 24a of the received employee code. In step S42, the extraction unit 13 accesses the personnel affairs data storage unit 21 and extracts personnel affairs item data of the personnel affairs record 21a of the received employee code. In step S43, the extraction unit 13 accesses the attendance data storage unit 22 and extracts attendance item data of the attendance record 22a of the received employee code. In step S44, the extraction unit 13 accesses the daily life data storage unit 27 and extracts item data of the daily life record 27a of the received employee code. In step S41 to step S44, the extraction unit 13 extracts predetermined item data that has been set in advance from each record and further extracts items selected by the user. All of the items extracted may be freely selected by the user. In addition, the predetermined item data that has been set in advance may be an item extracted, for example, by statistical analysis processing, which will be described later.

In step S45, the output control unit 14 of the management server 10 generates a report in the first output mode or a report in the second output mode as output data that is sent to the client terminal 3 requesting the report and sends the report to the client terminal 3. The client terminal 3 displays the report on a monitor or prints the report by using a printer to allow the employee to read the report. When a poor physical condition is found, this allows the employee to determine which one of the medical checkup items has a value worsened by the poor physical condition. The employee may also determine the cause of the worsening from the relationship with personnel affairs items and/or attendance items. Further, the employee may judge whether or not medical consultation is needed with reference to the values of the medical checkup items. In addition, the employee may seek the cause of the poor physical condition with reference to the attendance state.

Case where Person in Charge of Personnel Affairs Uses System

Case where Overwork and/or Poor Work Performance are Detected

With reference to Fig. 11, a description will be given of a case where the labor management system is used by a person in charge of personnel affairs and where overwork and/or poor work performance are detected.

In step S51, the extraction unit 13 accesses the stress check data storage unit 25 to judge whether or not an abnormal value is found in each check item data recorded in the stress check record 25a for each employee. The extraction unit 13 also accesses the attendance data storage unit 22 to judge whether or not an abnormal value is found in each attendance item data recorded in the attendance record 22a for each employee. The extraction unit 13 proceeds to step S52 when an abnormality is detected and ends the processing when no abnormality is detected.

In step S52, the extraction unit 13 extracts check item data containing the check item data in which an abnormality is detected. Further, in step S53, the extraction unit 13 extracts attendance item data containing the attendance item data in which an abnormality is detected. In step S54, the extraction unit 13 accesses the medical checkup data storage unit 24 and extracts main medical checkup items recorded in the medical checkup record 24a. In step S55, the extraction unit 13 accesses the personnel affairs data storage unit 21 and extracts each personnel affairs item data of the personnel affairs record 21a.

The extraction unit 13 may access the daily life data storage unit 27 and extract daily life data recorded in the daily life record 27a.

In step S56, the output control unit 14 generates a report in the first output mode or a report in the second output mode as output data that is sent to the client terminal 2 requesting the report and sends the report to the client terminal 2. The client terminal 2 displays the report on a monitor or prints the report by using a printer to allow the person in charge of personnel affairs to read the report. This allows the person in charge of personnel affairs to instantly find an employee who has an abnormality in the attendance item data and/or the check item data. The person in charge of personnel affairs may also easily determine whether or not the abnormality is related to the medical checkup results and/or the personnel affairs.

Case where Employee Returns from Leave of Absence

With reference to Fig. 12, a description will be given of a case where the labor management system is used by a person in charge of personnel affairs and where an employee returns from a leave of absence.

When the person in charge of personnel affairs operates the client terminal 2 and sends the management server 10, for example, the employee code of an employee who returns to work, in step S61, the extraction unit 13 accesses the stress check data storage unit 25 and extracts check item data recorded in the stress check record 25a. In step S62, the extraction unit 13 accesses the medical checkup data storage unit 24 and extracts main medical checkup items recorded in the medical checkup record 24a. In step S63, the extraction unit 13 accesses the attendance data storage unit 22 and extracts attendance item data recorded in the attendance record 22a. In step S64, the extraction unit 13 accesses the personnel affairs data storage unit 21 and extracts personnel affairs item data of the personnel affairs record 21a.

In step S65, the output control unit 14 generates a report in the first output mode or a report in the second output mode as output data that is sent to the client terminal 2 requesting the report and sends the report to the client terminal 2. The client terminal 2 displays the report on a monitor or prints the report by using a printer to allow the person in charge of personnel affairs to read the report. This allows the person in charge of personnel affairs to understand the stress state, health condition, personnel affairs, and attendance state of the returning employee before taking a leave of absence. The person in charge of personnel affairs may also use a report containing the stress state and/or health condition as a reference material for judging whether or not the work place is suitable for the returning employee and/or whether or not the system of assisting the employee is available.

Case where Performance improvement measures are Considered

With reference to Fig. 13, a description will be given of a case where the labor management system is used by a person in charge of personnel affairs and where performance improvement measures are considered.

When the person in charge of personnel affairs operates the client terminal 2 and sends the management server 10, for example, the employee code of an employee who is subject to the performance improvement measures, in step S71, the extraction unit 13 accesses the business performance data storage unit 26 and extracts business performance item data recorded in the business performance record 26a of the employee. The extraction unit 13 extracts, for example, sales amount, sales volume, ordered amount, and ordered volume of the employee in each month or in each year. In step S72, the extraction unit 13 accesses the medical checkup data storage unit 24 and extracts main medical checkup items recorded in the medical checkup record 24a. In step S73, the extraction unit 13 accesses the stress check data storage unit 25 and extracts check item data recorded in the stress check record 25a. In step S74, the extraction unit 13 accesses the attendance data storage unit 22 and extracts attendance item data recorded in the attendance record 22a. In step S75, the extraction unit 13 accesses the personnel affairs data storage unit 21 and extracts personnel affairs item data of the personnel affairs record 21a. In step S76, the extraction unit 13 accesses the payroll data storage unit 23 and extracts payroll item data of the payroll record 23a.

In step S77, the output control unit 14 generates a report in the first output mode or a report in the second output mode as output data that is sent to the client terminal 2 requesting the report and sends the report to the client terminal 2. The client terminal 2 displays the report on a monitor or prints the report by using a printer to allow the person in charge of personnel affairs to read the report. This allows the person in charge of personnel affairs to easily determine whether, for example, poor business performance of the employee is due to the stress state, health condition, personnel affairs, attendance state or salary. The person in charge of personnel affairs may also understand, for example, the health condition, stress state, and attendance state of an employee who has high business performance.

Case where Industrial Physician Uses System

Case where Consultation about Poor Physical Condition is Conducted

With reference to Fig. 14, a description will be given of a case where the labor management system is used by an industrial physician when conducting consultation, for example, about a poor physical condition.

When an industrial physician operates the client terminal 4 and sends the management server 10, for example, the employee code of an employee seeking consultation, in step S81, the extraction unit 13 accesses the medical checkup data storage unit 24 and extracts main medical checkup items recorded in the medical checkup record 24a. In step S82, the extraction unit 13 accesses the stress check data storage unit 25 and extracts check item data recorded in the stress check record 25a. In step S83, the extraction unit 13 accesses the attendance data storage unit 22 and extracts attendance item data recorded in the attendance record 22a. In step S84, the extraction unit 13 accesses the personnel affairs data storage unit 21 and extracts personnel affairs item data of the personnel affairs record 21a. In step S85, the extraction unit 13 accesses the daily life data storage unit 27 and extracts each item data of the daily life record 27a.

In step S86, the output control unit 14 generates a report in the first output mode or a report in the second output mode as output data which is sent to the client terminal 4 requesting the report and sends the report to the client terminal 2 requesting the report. The client terminal 4 displays the report on a monitor or prints the report by using a printer to allow the industrial physician to read the report. This allows the industrial physician to examine the employee seeking consultation through a medical interview with reference to the data including health condition, stress state, attendance, personnel affairs, and daily life of the employee. The industrial physician may seek the cause of the poor physical condition, for example, whether the cause is due to overtime work or late-night overtime work.

Case where High Stress, Overwork, and/or Poor Work Performance are Detected

Next, a description will be given of a case where the labor management system is used by an industrial physician and where high stress, overwork and/or poor work performance are detected. In this case, after the management server 10 performs processing similar to that described in Fig. 11, the output control unit 14 generates a report in the first output mode or a report in the second output mode as output data that is sent to the client terminal 4 requesting the report and sends the report to the client terminal 4. The client terminal 4 displays the report on a monitor or prints the report by using a printer to allow the industrial physician to read the report. This allows the industrial physician to instantly find an employee who has an abnormality in the attendance item data and/or the check item data. The industrial physician may also easily determine how the abnormality is related to the medical checkup results and/or the personnel affairs data. For example, whether or not stress produces a physical effect may be determined with reference to the medical checkup results. Also, whether or not self-care is appropriately taken may be determined by a medical interview.

According to the above embodiment, the following advantages are obtained.

(1) As shown in Fig. 6 and Fig. 7, the reports, which serve as output data, show medical checkup item data and/or check item data with other item data such as attendance item data so that the time axes are in conformance. This allows the operator to easily understand the correlation between these data. More specifically, in Fig. 6, the graphs of the item data are vertically aligned with the time axes in conformance. Therefore, the operator easily understands the correlation between the data. Further, in Fig. 7, the graphs of the item data are superimposed with the time axes in conformance. This facilitates understanding of the correlation between the data. The health of an employee may be managed by considering various aspects such as an attendance state and/or a status of personnel affairs status in addition to the medical checkup results and/or stress results.

(2) Further, the medical checkup item data and/or the check item data may also be displayed or printed in association with the personnel affairs data.

(3) As shown in Fig. 6, each graph of item data having a proper range further includes the line serving as an index showing the upper limit value and/or lower limit value. Thus, whether the item data is normal or abnormal is easily judged.

The above embodiment may also be carried out by being modified as follows, as appropriate.

The lines serving as indexes showing an upper limit value and a lower limit value of item data having a proper range may indicate one of the upper limit value and the lower limit value. Further, as shown in Fig. 15, an enhancement processing 46 may be performed on the item data showing an abnormality, for example, with a marker. The enhancement processing may be, for example, bold face lettering, italic lettering, underlined lettering, or colored lettering and is not particularly limited as long as the abnormal item data is shown more prominently than other item data. The enhancement processing may be performed in the second output mode shown in Fig. 7.

In the example shown in Fig. 7 as well, when a graph of "Total cholesterol" and "LDL cholesterol" are selected on the vertical axis, lines showing an upper limit value and/or a lower limit value of the proper range may be added.

A lower limit value line showing a lower limit value of the proper range may be added to each of the graphs shown in Fig. 6 and Fig. 15.

The format of output data may additionally include horizontally-arranged graphs. More specifically, an operator may be allowed to select one of vertically-arranged output, horizontally-arranged output, and superimposed output. Also, a combination of horizontally-arranged output and superimposed output may be output. Further, a combination of vertically-arranged output and horizontally-arranged output may be output. The output mode is not limited to the described three output modes.

When the time axis is given as a vertical axis, it is preferred that graphs are horizontally arranged so as to be orthogonal to the time axis.

Personal data such as the name, the date of birth, and/or the family structure of an employee may be added to the reports shown in Fig. 6, Fig. 7, and Fig. 15.

Data managed by the management server 10 is not limited to the data given in Fig. 2A to Fig. 2C and Fig. 3A to Fig. 3E. Further, item data configuring the data given in Fig. 2A to Fig. 2C and Fig. 3A to Fig. 3E is not limited to the data shown in the drawings.

As long as at least one of the medical checkup data and the stress check data is added to the report and the attendance data is selected as an additional item, other additional data is not particularly limited.

The management server 10 may be configured not to accumulate data in the data warehouse 30.

First Modified Example of Output Mode

Fig. 16 is a modified example of the first output mode (refer to Fig. 6) in which graphs of selected item data are vertically aligned. In Fig. 16, "Body weight," "Total cholesterol," and "LDL cholesterol" are selected, and the output control unit 14 generates a report which includes line graphs of "Body weight," "Total cholesterol," and "LDL cholesterol" extracted by the extraction unit 13 and a bar graph of "Total overtime hours" located below. In this example, "Transfer to different department," "Promotion," and "Job transfer" are indicated by icons 47. More specifically, with regard to "Transfer to different department," the icons 47 are shown at positions of the year 2009 and the year 2011. With regard to "Job transfer," the icon 47 is shown at a position of the year 2011. With regard to "Promotion," the icon 47 is shown at a position of the year 2012. Further, with regard to "Start of medication," the icon 47 is shown at a position of the year 2013. According to such an output mode, the use of the icon 47 for indication allows for larger presentation of a line graph and/or a bar graph than the example shown in Fig. 6 and also allows a larger number of items to be shown on the graphs. When color is specified for each item so that the items have different colors, the icons 47 are easier to be seen. Different colors may be used, for example, such that an icon of the item related to attendance is yellow and an icon related to medical data is red.

Second Modified Example of Output Mode

Fig. 17 is a modified example of the second output mode (refer to Fig. 7) in which graphs of selected item data are superimposed. In the example shown in Fig. 17 as well, "Transfer to different department," "Promotion," "Job transfer," and "Start of medication" are indicated by the icons 47.

Third Modified Example of Output Mode

Fig. 18 is a modified example of an output mode in which graphs of item data are superimposed for each large category. As shown in Fig. 18, the output control unit 14 superimposes data of subcategories within a large category and shows the data for each large category, namely, "Medical checkup results," "Attendance," and "Daily life" for a period of time from the year 2009 to the year 2013. Specifically, with regard to "Medical checkup results," the line graphs of BMI, abdominal circumference, systolic blood pressure, diastolic blood pressure, blood sugar, and HDL cholesterol are shown in a superimposed manner with the time axes in conformance. Further, with regard to "Attendance," the bar graphs of the overtime hours and the holiday work are shown with the time axes in conformance. With regard to "Daily life," the bar graphs of an amount of exercise and hours of sleep are shown with the time axes in conformance.

In this output mode, in the superimposed graph presentation of each large category, each of the subcategory data is shown by converting the value of the subcategory data so that the lower limit value or minimum value of the subcategory is set to zero and the upper limit value or maximum value is set to ten.

Further, in this output mode as well, the icons 47 for "Transfer to different department," "Promotion," "Job transfer," and "Start of medication" are shown.

Fourth Modified Example of Output Mode

In the output modes shown in Fig. 6, Fig. 7, and Fig. 15 to Fig. 18, for example, a unit of display of "Total overtime hours" is a yearly basis. Instead, the unit of display may be switched to a unit of, for example, one month, three months, six months, and one year. Fig. 19 shows an example in which "Total overtime hours" is switched from yearly basis to monthly basis.

Fifth Modified Example of Output Mode

Fig. 20A is a modified example of an output mode in which graphs of selected item data are superimposed and also icons are used. In Fig. 20A, data for a period of time from the year 2013 to the year 2017 is extracted. At an upper section, overtime hours are shown by using bar graphs. At a lower section thereof, stress check results are shown by a five-grade evaluation. The stress check results may be evaluated according to a five-grade evaluation or a two-grade evaluation selected by a radio button 61. The number of grades in evaluation is not particularly limited, and the results may be evaluated according to a three-grade evaluation or a ten-grade evaluation. The number of grades in evaluation may be selected from the options. A remarks column is provided at a lower section of the stress check results in which the use of paid leave, a medical checkup date, and/or a date of interview with a public health nurse are indicated. At a lower section of the remarks column, the medical checkup results are shown by a line graph in a superimposed manner. Here, the medical checkup results shows triglyceride, blood sugar levels, and uric acid values. On the vertical axis of the line graph, the lower limit value or minimum value of each item is set to zero and the upper limit value or maximum value is set to ten to convert values of each item data. The vertical axis is provided with evaluation references of the medical checkup results. The evaluation references include five grades from A to E where A corresponds to being normal, B corresponds to mild abnormality, C corresponds to medical follow-up, D corresponds to medical treatment needed, and E corresponds to under treatment. In the graph, the range of each grade is indicated in a band shape in parallel with the horizontal axis. The evaluation reference of the medical checkup results may be selected by using a radio button 62 from the evaluation reference of the Japan Society of Ningen Dock and the internal evaluation reference. Here, the evaluation reference of the Japan Society of Ningen Dock is selected. Further, when a medical examination is conducted using, for example, images and/or videos such as a chest X-ray, a stomach X-ray, MRI examinations of the brain, and/or ultrasound scanning of the liver, an X-ray button 64, an MRI button 65, and an ultrasound scanning button 66 are shown in the year in which the medical examination was conducted. When the X-ray button 64 is pressed, image data of the X-ray is shown. When the MRI button 65 is pressed, image data of MRI is shown. When the ultrasound scanning button 66 is pressed, video data of ultrasound scanning is reproduced and shown. The X-ray button 64, the MRI button 65, and the ultrasound scanning button 66 may be shown only in the year in which an abnormality is found. Alternatively, once an abnormality is found, any of the buttons 64, 65 and 66, corresponding to the medical examination finding the abnormality may be shown in years thereafter.

Further, at a lower section of the medical checkup results, changes in the number of walking steps are shown as daily life data.

Still further, smoking amounts (the number cigarettes per day) and/or alcohol intake amounts are shown by icons 63 as medical interview data.

Fig. 20B shows a list of the icons 63 that may be used in the output mode shown in Fig. 20A.

Statistical Analysis Processing 1

In the data warehouse 30, statistical analysis processing is performed for selecting the data to be displayed on an output screen shown in Fig. 6, Fig. 7, and Fig. 15 to Fig. 20 so that medical checkup results and/or stress check results are displayed with data highly related to these results such as attendance data.

Specifically, the analysis unit 32 of the data warehouse 30 performs a statistical analysis on the data accumulated in the accumulation unit 31 in the following manner. Fig. 21 is a flowchart showing procedures of the statistical analysis processing 1. Specifically, the correlation is calculated between a first target item included in a first target item group of any one of an item group of personnel affairs data, an item group of attendance data, an item group of payroll data, an item group of medical checkup data, an item group of stress check data, an item group of business performance data, an item group of daily life data, and an item group of medical data that are accumulated in the accumulation unit 31 and a second target item included in a second target item group that is one of the item groups other than the first target item group.

In step S101, the analysis unit 32 of the data warehouse 30 sets a unit of analysis for performing a statistical analysis. The analysis unit 32 sets a period of time that is to be analyzed, for example, one month, three months, six months, one year, and so on.

In step S102, the analysis unit 32 sets a first target item to calculate a correlation coefficient of a second target item with respect to the first target item. The correlation coefficient is calculated based on first target data of the first target item and second target data of the second target item, which are accumulated in the accumulation unit 31. The correlation coefficient is given as a real-number value between -1 and 1. When the value is close to 1, a positive correlation is found between two random variables. When it is close to -1, a negative correlation is found. When the value is close to zero, no correlation is found.

The analysis unit 32 sets a first target item, sets a second target item with respect to the first target item, and calculates a correlation coefficient for each unit of analysis based on first target data of the first target item and second target data of the second target item, which are accumulated in the accumulation unit 31. The analysis unit 32 also calculates a correlation coefficient of target items having a one-to-one relationship, that is, a correlation coefficient between a single first target item and a single second target item. The analysis unit 32 calculates a correlation coefficient of all combinations of the first target items and the second target items.

For example, a relationship between a first target item and a second target item includes the following.

(i) Each item in the item group of the medical checkup data is selected as a first target item. Also, each item in the item group of the attendance data, each item in the item group of the personnel affairs data, each item in the item group of the stress check data, each item in the item group of the payroll data, each item in the item group of the daily life data, and each item in the item group of the medical data are selected as a second target item. Then, the correlation coefficient of each combination is calculated.

(ii) Each item in the item group of the stress check data is selected as a first target item. Each item in the item group of the medical checkup data, each item in the item group of the attendance data, each item in the item group of the personnel affairs data, each item in the item group of the payroll data, each item in the item group of the daily life data, and each item in the item group of the medical data are selected as a second target item. Then, the correlation coefficient of each combination is calculated.

(iii) Each item in the item group of the business performance data is selected as a first target item. Each item in the item group of the medical checkup data, each item in the item group of the attendance data, each item in the item group of the personnel affairs data, each item in the item group of the stress check data, each item in the item group of the payroll data, each item in the item group of the daily life data, and each item in the item group of the medical data are selected as a second target item. Then, the correlation coefficient of each combination is calculated.

With regard to items such as post advancement, demotion, assignment to another company, release from assignment to another company, transfer without family, end of transfer without family, leave of absence, return to work, marriage, divorce, and childbirth in the item group of the personnel affairs data, occurrence and non-occurrence of each event are used as data.

In step S103, the analysis unit 32 extracts a second target item highly correlated with a first target item as a related item of the first target item. The analysis unit 32 stores a first threshold value as the threshold value of a positive correlation in a storage unit such as a memory and stores a second threshold value as the threshold value of a negative correlation in a storage unit such as a memory. When calculating the correlation coefficient of a second target item with respect to a first target item, the analysis unit 32 extracts a second target item having a correlation coefficient greater than the first threshold value and a second target item having a correlation coefficient less than the second threshold value as highly-correlated related items.

Alternatively, in another example, the analysis unit 32 judges whether or not the absolute value of a calculated correlation coefficient is greater than a threshold value stored in the storage unit such as a memory and extracts a second target item having a correlation coefficient, the absolute value of which is greater than the threshold value, as a highly-correlated related item.

For example, in the examples (i) to (iii) described above, the following second target items are highly correlated with the first target item and extracted as a related item.

(i) When the first target item is BMI in the item group of the medical checkup data, overtime hours in the item group of the attendance data, marriage and/or transfer without family in the item group of the personnel affairs data, work load in the item group of the stress check data, and an amount of exercise, consumed calories, and/or intake calories in the item of the daily life data are extracted as related items.

(ii) When the first target item is comprehensive determination in the item group of the stress check data, overtime hours and/or the number of days of holiday work in the item group of the attendance data, transfer to a different department in the item group of the personnel affairs data, body weight and/or blood pressure in the item group of the medical checkup data, and blood pressure, the number of walking steps, and/or hours of sleep in the item group of the daily life data are extracted as related items.

(iii) When the first target item is sales amount in the item group of the business performance data, overtime hours and/or the number of days off in the item group of the attendance data, total cholesterol in the item group of the medical checkup data, comprehensive determination in the item group of the stress check data, and hours of sleep in the item group of the daily life data are extracted as related items.

When the analysis unit 32 extracts a second target item serving as the related item of the first target item, in step S104, the registration unit 33 gives identification data to a combination of the first target item and the second target item and registers the combination in a memory. For example, the related item of each medical checkup item used for determining lifestyle disease is registered as a related item of lifestyle disease. For example, a diagnosis of metabolic syndrome is determined by abdominal circumference, BMI, systolic blood pressure, diastolic blood pressure, blood sugar, and HDL cholesterol. The registration unit 33 sets each of abdominal circumference, BMI, systolic blood pressure, diastolic blood pressure, blood sugar, and HDL cholesterol as the first target item. Then, each of abdominal circumference, BMI, systolic blood pressure, diastolic blood pressure, blood sugar, and HDL cholesterol is associated with a second target item serving as the related item. The registration unit 33 registers, as the specification of metabolic syndrome, combinations of each first target item related to metabolic syndrome and the second target item serving as the related item associated with the first target item. The management server 10 obtains the specification from the registration unit 33 and also obtains item data of the first target item and item data of the second target item in a specification selected by operation of an employee from each of the storage units 21 to 28 to output a personal report, for example, in a format similar to those of Fig. 6, Fig. 7, and Fig. 15 to Fig. 20.

Case where Employee Sees Medical Checkup Results

Fig. 22 is a flowchart showing procedures of a case where an employee sees medical checkup results.

In step S111, the employee operates the client terminal 3 and sends, for example, the employee code from the client terminal 3 to the management server 10. If the employee wants to know information related to metabolic syndrome, the employee selects the specification of metabolic syndrome registered by the registration unit 33 and sends the specification to the management server 10.

In step S112, the extraction unit 13 of the management server 10 extracts data of first target items in accordance with the received specification. For example, when metabolic syndrome is selected, item data of BMI, which is related to metabolic syndrome, is extracted as a first target item. Similarly, the extraction unit 13 extracts item data of abdominal circumference, systolic blood pressure, diastolic blood pressure, blood sugar, and HDL cholesterol as first target items.

In step S113, the extraction unit 13 extracts second target items as related items of each first target item. For example, item data of overtime hours in a target item group of attendance data, marriage and/or transfer without family in a target item group of personnel affairs data, work load in a target item group of stress check data, and amount of exercise, consumed calories, and/or intake calories in a target item group of daily life data is information related to BMI, which serves as the first target item, and is extracted.

Further, items selected by the user may be extracted.

In step S114, the output control unit 14 of the management server 10 generates a report as output data that is sent to the client terminal 3 requesting the report. The output control unit 14 sends the report to the client terminal 3 in the requested output mode. The client terminal 3 displays the report in a format similar to those of Fig. 6, Fig. 7, and Fig. 15 to Fig. 20 on a monitor or prints the report by using a printer to allow the employee to read the report.

Fig. 23 is a diagram showing an output mode of the report.

In the report 41 of Fig. 23, graphs of the selected item data are shown in a superimposed manner. In the report 41, a combination pattern may be selected with the pull-down menu. In the first pull-down menu 43, "Check stress check results" may be selected in addition to "Check medical checkup results" and other items. Here, "Metabolic syndrome," which is the specification selected by the user, is selected. In addition, the second pull-down menu 44 is provided for selecting target data to be displayed. In the second pull-down menu 44, a designated item of large categories may be selected from "Attendance," "Personnel affairs," "Stress check results," "Medical checkup," and "Daily life." Then, with regard to the selected large category, item data may further be selected as a subcategory of the designated item. In the example shown in Fig. 23, "Attendance," "Personnel affairs," "Stress check," "Medical checkup," and "Daily life" are selected in accordance with "Metabolic syndrome," which is selected. The output control unit 14 accesses the personnel affairs data storage unit 21, the attendance data storage unit 22, the medical checkup data storage unit 24, the stress check data storage unit 25, and the daily life data storage unit 27 to extract data of the selected subcategory using the extraction unit 13 and adds the data to the report 41. In the report 41, the position of the graph of each item is aligned in conformance on the time axis, and further, the graphs are superimposed. Thus, each data on metabolic syndrome may be checked not only with reference to the medical checkup results but also together with items such as personnel affairs and/or attendance.

As shown in Fig. 23, in a state that the report 41 is displayed on a monitor of the client terminal 3, when an operation portion such as a mouse is used to select the vertically-arranged display with the radio button 42, the output control unit 14 generates output data in a newly-selected output mode of the vertically-arranged display. Consequently, the output data in the newly-generated output mode is displayed on the monitor of the client terminal 3.

Statistical Analysis Processing 2

The analysis unit 32 of the data warehouse 30 performs a statistical analysis on the data accumulated in the accumulation unit 31 in the following manner. Specifically, the analysis unit 32 uses a multiple regression analysis to calculate a correlation of a first target item of any one of first target item groups including an item group in personnel affairs data, an item group in attendance data, an item group in payroll data, an item group in medical checkup data, an item group in stress check data, an item group in business performance data, an item group in daily life data, and an item group in medical data with a second target item of the second target item group, which are accumulated in the accumulation unit 31. The analysis unit 32 also generates a relational expression showing a relationship between the first target item and the second target item.

Fig. 24 is a flowchart showing procedures of the statistical analysis processing 2.

In step S121, the analysis unit 32 of the data warehouse 30 sets a unit of analysis for performing a statistical analysis. The analysis unit 32 sets a period of time that is to be analyzed, for example, one month, three months, six months, one year, and so on.

In step S122, the analysis unit 32 sets a first target item (objective variable), sets a second target item (explanatory variable) with respect to the first target item, and performs a multiple regression analysis for each unit of analysis based on the first target data of first target items and the second target data of second target items, which are accumulated in the accumulation unit 31, to calculate a t value between the first target item (objective variable) and the second target item (explanatory variable). The analysis unit 32 calculates t values of a plurality of second target items (explanatory variables) for a single first target item (objective variable). The t value is a value indicating that the larger the absolute value is, the larger the effects become.

Specific examples of relationships between the first target item and the second target item are as follows.

(i) When the first target item (objective variable) corresponds to each item in the item group of the medical checkup data, the second target item (explanatory variable) corresponds to each item in the item group of the attendance data, each item in the item group of the personnel affairs data, each item in the item group of the stress check data, each item in the item group of the payroll data, each item in the item group of the daily life data, and each item in the item group of the medical data.

(ii) When the first target item (objective variable) corresponds to each of the stress check items in the item group of the stress check data, the second target item (explanatory variable) corresponds to each item in the item group of the medical checkup data, each item in the item group of the attendance data, each item in the item group of the personnel affairs data, each item in the item group of the payroll data, each item in the item group of the daily life data, and each item in the item group of the medical data.

(iii) When the first target item (objective variable) corresponds to each item in the item group of the business performance data, the second target item (explanatory variable) corresponds to each item in the item group of the medical checkup data, each item in the item group of the attendance data, each item in the item group of the personnel affairs data, each item in the item group of the stress check data, each item in the item group of the payroll data, each item in the item group of the daily life data, and each item in the item group of the medical data.

In step S123, when a t value obtained by the multiple regression analysis does not meet conditions stored in the storage unit such as a memory, the analysis unit 32 removes the corresponding second target item (explanatory variable) from the target. Then, the analysis unit 32 again performs the multiple regression analysis. Specifically, the analysis unit 32 removes an explanatory variable having a t value such that $t^2 < 2$ from the target and again performs the multiple regression analysis. The analysis unit 32 repeats the multiple regression analysis until the t values of all explanatory variables are $t^2 \geq 2$. This removes second target items having a low correlation with the first target item from the target. The remaining second target items serve as the related items.

In step S124, when the t values of all explanatory variables are $t^2 \geq 2$, the analysis unit 32 obtains a slope and an intercept from the results of the multiple regression analysis and generates a relational expression of the objective variable and the explanatory variable.

Fig. 25 is a diagram showing the multiple regression analysis.

Here, X1 to X8 are explanatory variables and Y is an objective variable. In the first multiple regression analysis, the t values of the explanatory variables X1, X5 and X6 are $t^2 < 2$ and thus the explanatory variables X1, X5 and X6 are removed. In the second multiple regression analysis, the t value of the explanatory variable X7 is $t^2 < 2$ and thus the explanatory variable X7 is removed. In the third multiple regression analysis, the t values of all explanatory variables X2, X3, X4 and X8 are $t^2 \geq 2$ and thus extracted as related items.

Further, the following expression is generated as a relational expression.

$$Y = aX2 + bX3 + cX4 + dX8 + e$$

where a, b, c, and d are slopes, and e is an intercept.

When the analysis unit 32 extracts a second target item as a related item of a first target item, in step S124, the registration unit 33 registers a combination of the first target item and the second target item and a relational expression in the storage unit such as a memory. The analysis unit 32 uses the data accumulated in the data warehouse 30 to generate relational expressions of the second target item (explanatory variable) for various first target items (objective variables) and register the expressions in the storage unit.

Personal Future Projection

Fig. 26 is a flowchart of processing that performs personal future projection of an employee.

The analysis unit 32 projects a future value of a first target item (objective variable) of each employee according to the relational expression generated in Fig. 23 and Fig. 24. In step S131, the analysis unit 32 selects a first target item (objective variable Y) of an employee undergoing the future projection, for example, from a list. The analysis unit 32 selects any of an item in the item group of the medical checkup data, an item in the item group of the stress check data, and an item in the item group of the business performance data. Then, the analysis unit 32 sets a relational expression of the selected first target item (objective variable).

In step S132, the analysis unit 32 extracts a second target item (explanatory variable X) used in the set relational expression.

In step S133, the analysis unit 32 designates a specified day (day/month/year) for which the future projection is performed. The analysis unit 32 uses a least squares method to calculate a value of the second target item (explanatory variable X) on the specified future day.

In step S133, the analysis unit 32 applies the value of the second target item (explanatory variable X) calculated in step S124 to a calculation expression to calculate the first target item (objective variable Y).

The extraction unit 13 of the management server 10 obtains the second target item (explanatory variable X) and/or the first target item (objective variable Y) calculated by the analysis unit 32 and reports the future value of the first target item (objective variable Y) or comments of, for example, an expert based on the value to the employee in a report of the format as shown in Fig. 23.

According to the above-described statistical analysis processing 1 and 2, the following advantages are obtained.

(1) As shown in Fig. 21, since the analysis unit 32 calculates the correlation of a second target item with a first target item and registers a highly-correlated second target item as a related item, the report 41 includes data on the related item of the first target item. This allows, for example, the employee to know a specific medical checkup item and a related item that is highly related to the specific medical checkup item in association with each other.

(2) When related items are extracted, a period of time is designated as a unit of analysis. This provides the degree of freedom for extracting related items. Thus, an unnecessarily long period of time does not need to be set in processing of extracting related items.

(3) Related items of the first target item may be extracted from many target item groups such as the item group of the personnel affairs data and/or the item group of the business performance data in addition to the item group of the medical checkup data, the item group of the stress check data, and the item group of the attendance data. More specifically, the range from which the related items of the first target item are extracted may be widened. Thus, a second target item that is highly correlated with the first target item is selected from a wide range of items.

(4) As shown in Fig. 23 and Fig. 24, a highly-correlated explanatory variable is extracted by a multiple regression analysis.

(5) The generation of the relational expression allows for future projection of the first target item (objective variable) for each employee. Further, the projection result may be reported to the employee.

(6) A report including a first target item and a related item highly correlated with the first target item is issued to an employee. When seeing the report, the employee may easily understand the relationship between the first target item and the related item.

(7) In the report, the first target item and the related item are displayed with the time axes in conformance. This facilitates understanding of the correlation between the items.

The above-described statistical analysis processing 1 and 2 may also be carried out as follows, as appropriate.

The report may be obtained not only by an employee but also by a person in charge of personnel affairs and/or an industrial physician by operating the corresponding terminal.

The report may be displayed not only in the form of a graph but also may be displayed as a list.

Setting of the unit of analysis may be omitted.

The analysis unit 32 and/or the registration unit 33 may be installed on the management server 10. Further, the management server 10 may also have the function of the accumulation unit 31 of the data warehouse 30.

## Claims

1. A labor management system comprising:

   a first item group including at least one of an attendance item group and a personnel affairs item group;
   a second item group including at least one of a medical checkup item group and a stress check item group;
   first data showing a result of each of first items that configure the first item group, wherein the first data is data that shows the result of each of first items of an employee as a history;
   second data showing a result of each of second items that configure the second item group, wherein the second data is data that shows the result of each of second items of the employee as a history;
   a storage unit that stores the first data and the second data;
   an input unit in which at least one of the first items and at least one of the second items are respectively input as designated items; and
   an output control unit that controls output to an output unit so that the output unit outputs a graph showing a result of each of the designated items, wherein the graph includes the first data and the second data stored in the storage unit, and the result of each of the designated items is positioned on a time axis so that the designated items are in conformance.

2. The labor management system according to claim 1, wherein
   the output control unit controls the output so that the output unit outputs a graph for each of the designated items and also each of the graphs has a time axis that is consistent in range.

3. The labor management system according to claim 2, wherein
   the output control unit controls the output so that the graphs are aligned with each other in a direction orthogonal to the time axis.

4. The labor management system according to claim 1, wherein the output control unit controls the output so that the results of the designated items are superimposed on one another in a single graph.

5. The labor management system according to any one of claims 1 to 4, wherein the output control unit has a proper range for the result of each of the second items and further adds an index showing whether or not the result of the second item is within the proper range to a graph showing the result of the second item.

6. The labor management system according to any one of claims 1 to 5, further comprising an analysis unit that analyzes, among a first item configuring the first item group and a second item configuring the second item group, whether or not a correlation of one item with the other item satisfies a condition indicating height of the correlation,
   wherein when the condition is satisfied, the analysis unit extracts the one item as a related item of the other item.

7. A labor management method comprising:

   specifying at least one of an attendance item group and a personnel affairs item group as a first item group;
   specifying at least one of a medical checkup item group and a stress check item group as a second item group;
   specifying first data showing a result of each of first items that configure the first item group, wherein the first data is data showing the result of each of the first items of an employee as a history;
   specifying second data showing a result of each of second items that configure the second item group, wherein the second data is data showing the result of each of the second items of the employee as a history;
   storing the first data and the second data in a storage unit;
   inputting at least one of the first items and at least one of the second items respectively as designated items with an input unit; and
   controlling an output to an output unit with an output control unit so that the output unit outputs a graph showing a result of each of the designated items, wherein the graph includes the first data and the second data stored in the storage unit, and the result of each of the designated items is positioned on a time axis so that the designated items are in conformance.

8. A labor management program comprising:

specifying at least one of an attendance item group and a personnel affairs item group as a first item group;
specifying at least one of a medical checkup item group and a stress check item group as a second item group;
specifying first data showing a result of each of first items that configure the first item group, wherein the first data is data showing the result of each of the first items of an employee as a history;
specifying second data showing a result of each of second items that configure the second item group, wherein the second data is data showing the result of each of the second items of the employee as a history;
having a computer function as a means for storing the first data and the second data in a storage unit;
inputting at least one of the first items and at least one of the second items respectively as designated items with an input unit; and
having a computer function as a means for having an output control unit control an output unit so that the output unit outputs a graph showing a result of each of the designated items, wherein the graph includes the first data and the second data stored in the storage unit, and the result of each of the designated items is positioned on a time axis so that the designated items are in conformance.

**9.** A labor management system comprising:

an accumulation unit that accumulates data showing a result of each of items that configure an item group of attendance data, an item group of medical checkup data, and an item group of stress check data together with a history;
a first target item group that is one of the item group of the attendance data, the item group of the medical checkup data, and the item group of the stress check data;
a second target item group that is at least one item group other than the first target item group;
first target data that is data showing a result of each of first target items that configure the first target item group as a history;
second target data that is data showing a result of each of second target items that configure the second target item group as a history; and
an analysis unit that extracts the second target item as a related item when a correlation of the second target item with the first target item satisfies a condition indicating height of the correlation.

**10.** The labor management system according to claim 9, wherein the analysis unit sets a period of time serving as a unit of analysis to extract the second target item as the related item during the set period of time.

**11.** The labor management system according to claim 9 or 10, wherein with regard to at least one item group of an item group of personnel affairs data and an item group of business performance data, the accumulation unit further accumulates data showing a result of each of items that configure the item group as a history.

**12.** The labor management system according to any one of claims 9 to 11, wherein the analysis unit uses a multiple regression analysis to remove the second target item that does not satisfy the condition from the related item.

**13.** The labor management system according to claim 12, wherein with regard to the second target item extracted as the related item, the analysis unit calculates the second target data on a specified future day and calculates the first target data on the specified day based on the calculated second target data.

**14.** The labor management system according to any one of claims 9 to 13, further comprising an output control unit that controls output to an output unit so that the output unit outputs first target data of the first target item and second target data of the second target item extracted as the related item.

**15.** A labor management method comprising:

accumulating data that shows a result of each of items that configure an item group of attendance data, an item group of medical checkup data, and an item group of stress check data together with a history in an accumulation unit;
specifying one of the item group of the attendance data, the item group of the medical checkup data, and the item group of the stress check data as a first target item group;
specifying at least one item group other than the first target item group as a second target item group;
specifying first target data that is data showing a result of each of first target items that configure the first target item group as a history;
specifying second target data that is data showing a result of each of second target items that configure the second target item group as a history; and
extracting the second target item as a related item with an analysis unit when a correlation of the second target item with the first target item satisfies a condition indicating height of the correlation.

**16.** A labor management program comprising:

having a computer function as a means for accumulating data that shows a result of each of items that configure an item group of attendance data, an item group of medical checkup data, and an item group of stress check data together with a history in an accumulation unit;
specifying one of the item group of the attendance data, the item group of the medical checkup data, and the item group of the stress check data as a first target item group;
specifying at least one item group other than the first target item group as a second target item group;
specifying first target data that is data showing a result of each of first target items that configure the first target item group as a history;
specifying second target data that is data showing a result of each of second target items that configure the second target item group as a history; and
having a computer function as a means for having an analysis unit extract the second target item as a related item when a correlation of the second target item with the first target item satisfies a condition indicating height of the correlation.

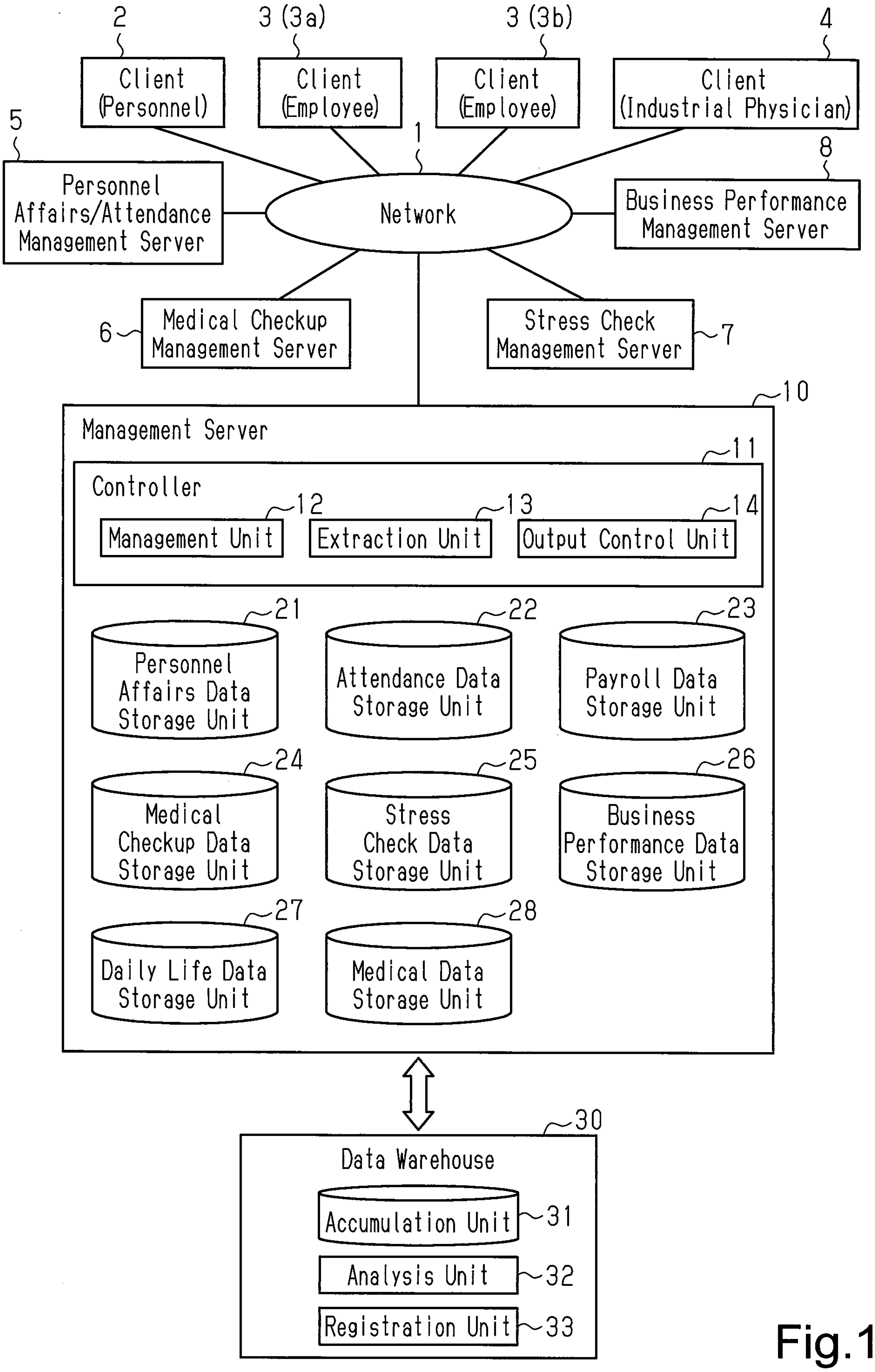
2
3 (3a)
3 (3b)
4
Client (Personnel)
Client (Employee)
Client (Employee)
Client (Industrial Physician)
5
1
8
Personnel Affairs/Attendance Management Server
Network
Business Performance Management Server
6
Medical Checkup Management Server
Stress Check Management Server
7
10
Management Server
11
Controller
12
13
14
Management Unit
Extraction Unit
Output Control Unit
21
22
23
Personnel Affairs Data Storage Unit
Attendance Data Storage Unit
Payroll Data Storage Unit
24
25
26
Medical Checkup Data Storage Unit
Stress Check Data Storage Unit
Business Performance Data Storage Unit
27
28
Daily Life Data Storage Unit
Medical Data Storage Unit
30
Data Warehouse
Accumulation Unit
31
Analysis Unit
32
Registration Unit
33

Fig.1

Fig.2A

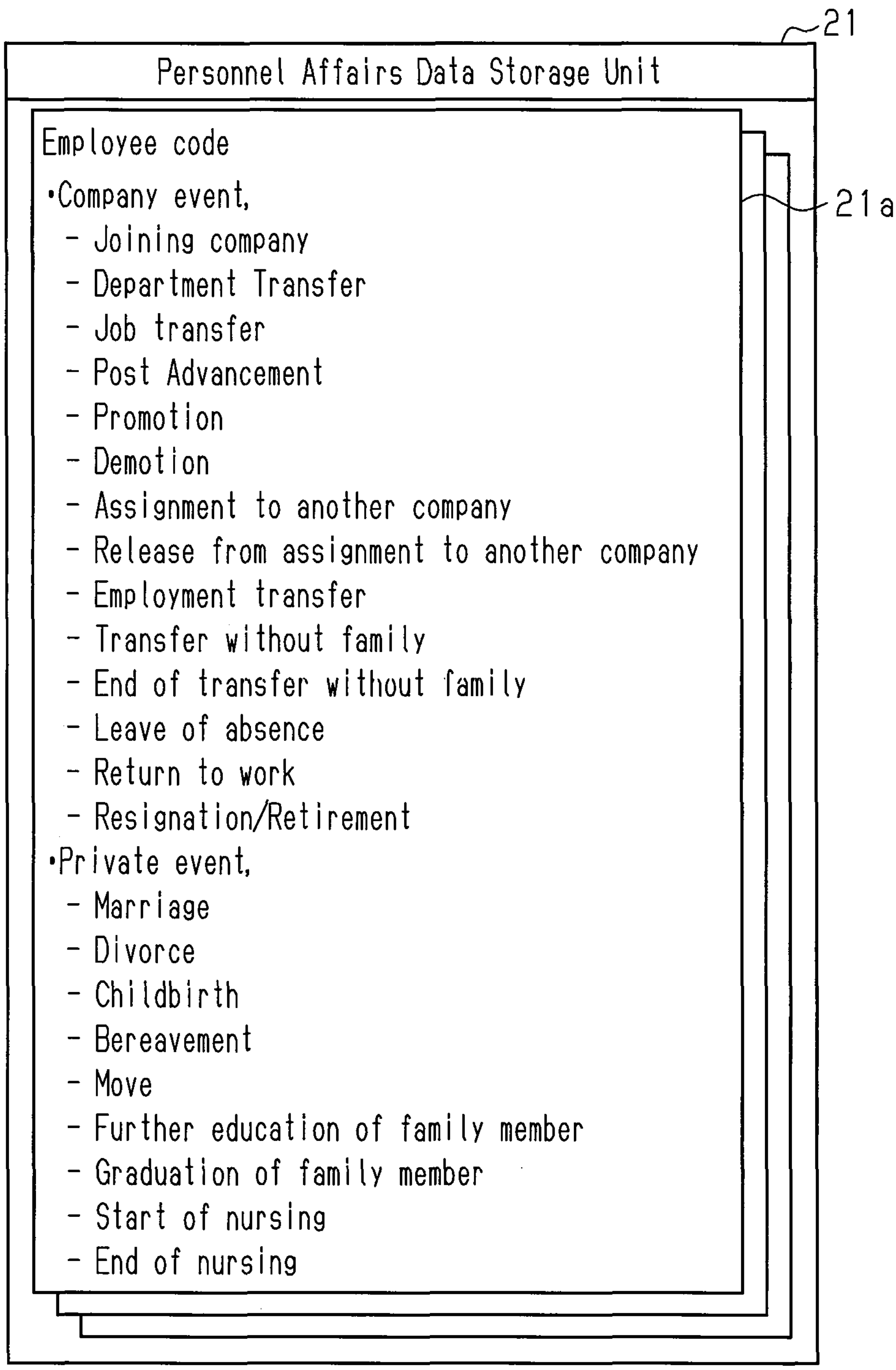
21
Personnel Affairs Data Storage Unit
21a
Employee code
•Company event,
- Joining company
- Department Transfer
- Job transfer
- Post Advancement
- Promotion
- Demotion
- Assignment to another company
- Release from assignment to another company
- Employment transfer
- Transfer without family
- End of transfer without family
- Leave of absence
- Return to work
- Resignation/Retirement
•Private event,
- Marriage
- Divorce
- Childbirth
- Bereavement
- Move
- Further education of family member
- Graduation of family member
- Start of nursing
- End of nursing

Fig.2B

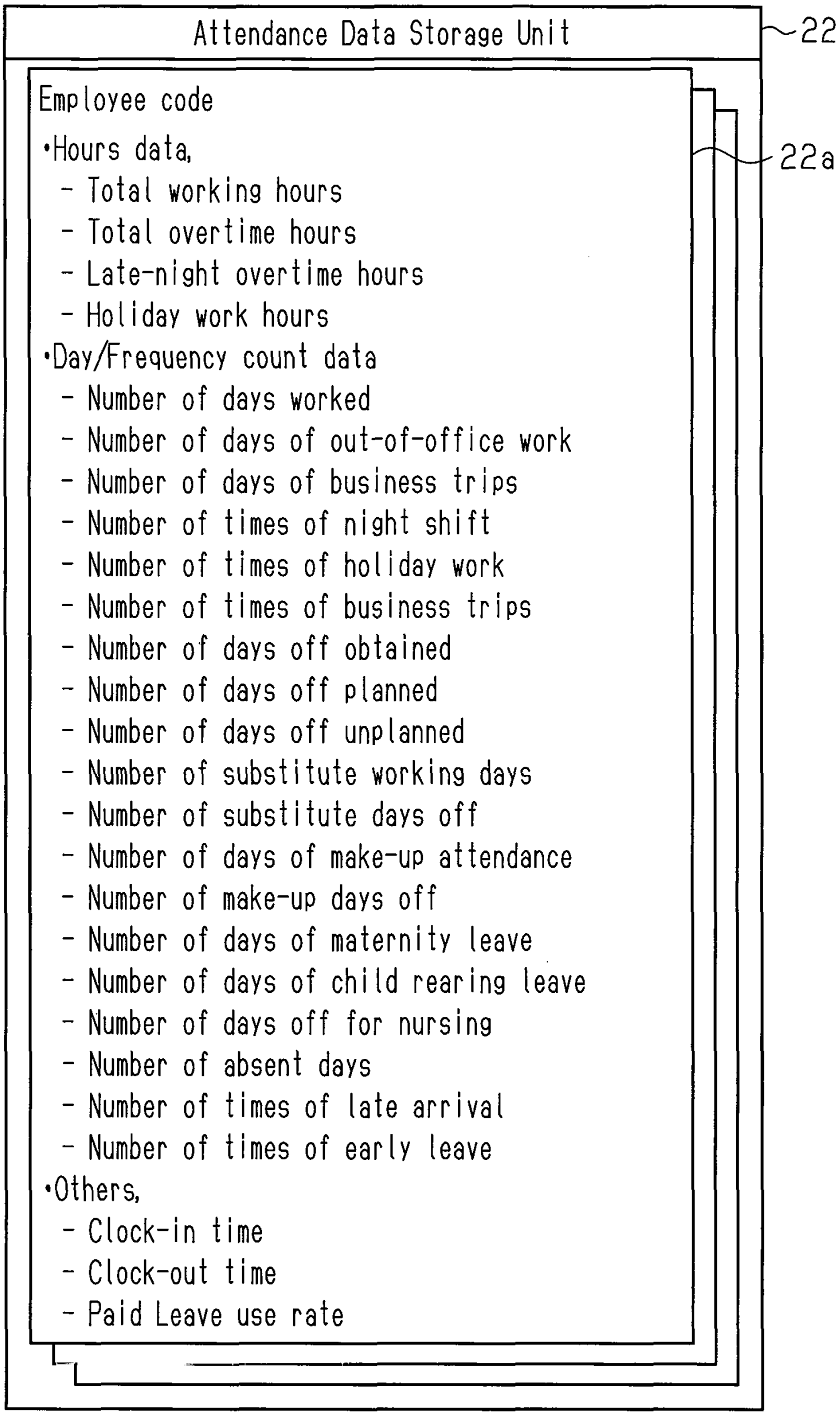
Attendance Data Storage Unit
22
22a
Employee code
•Hours data,
- Total working hours
- Total overtime hours
- Late-night overtime hours
- Holiday work hours
•Day/Frequency count data
- Number of days worked
- Number of days of out-of-office work
- Number of days of business trips
- Number of times of night shift
- Number of times of holiday work
- Number of times of business trips
- Number of days off obtained
- Number of days off planned
- Number of days off unplanned
- Number of substitute working days
- Number of substitute days off
- Number of days of make-up attendance
- Number of make-up days off
- Number of days of maternity leave
- Number of days of child rearing leave
- Number of days off for nursing
- Number of absent days
- Number of times of late arrival
- Number of times of early leave
•Others,
- Clock-in time
- Clock-out time
- Paid Leave use rate

Fig.2C

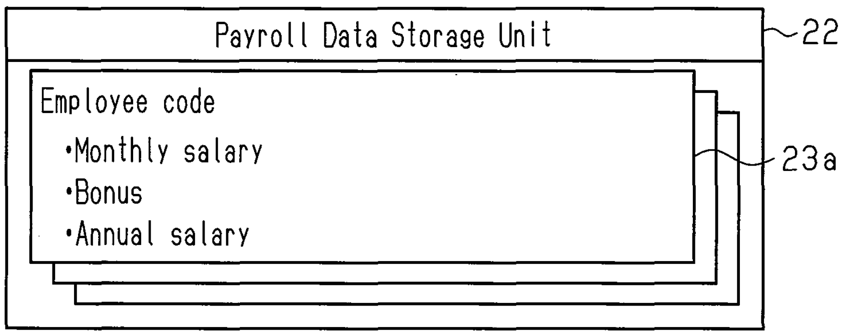
Payroll Data Storage Unit
22
23a
Employee code
•Monthly salary
•Bonus
•Annual salary

Fig.3A

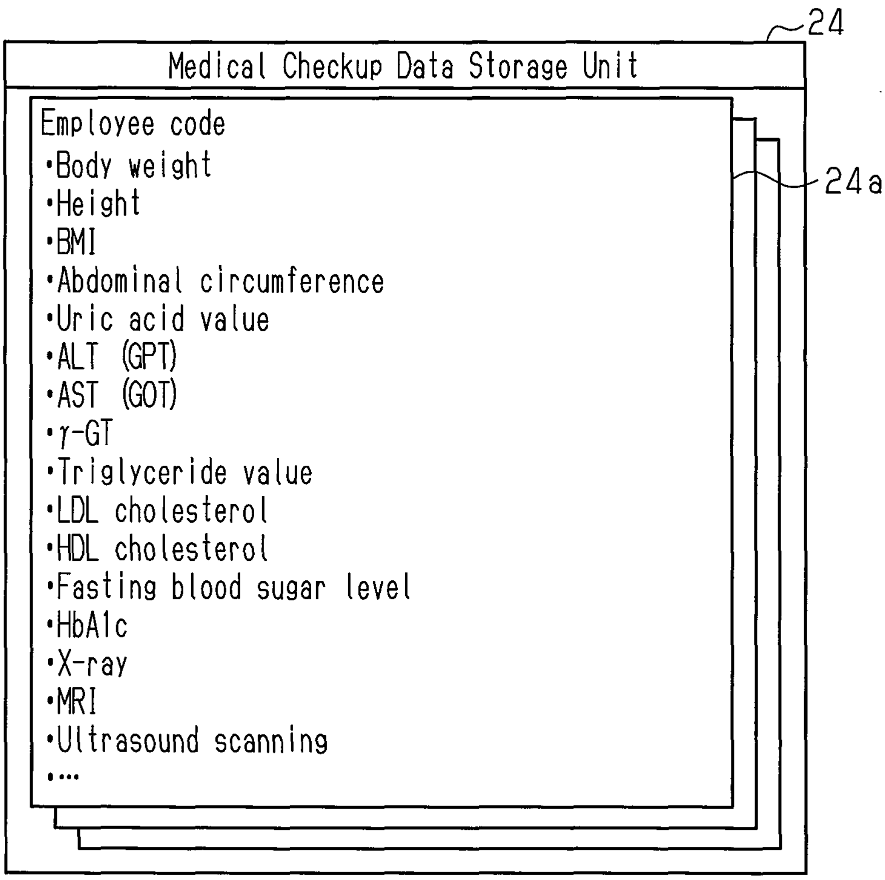


Fig.3B

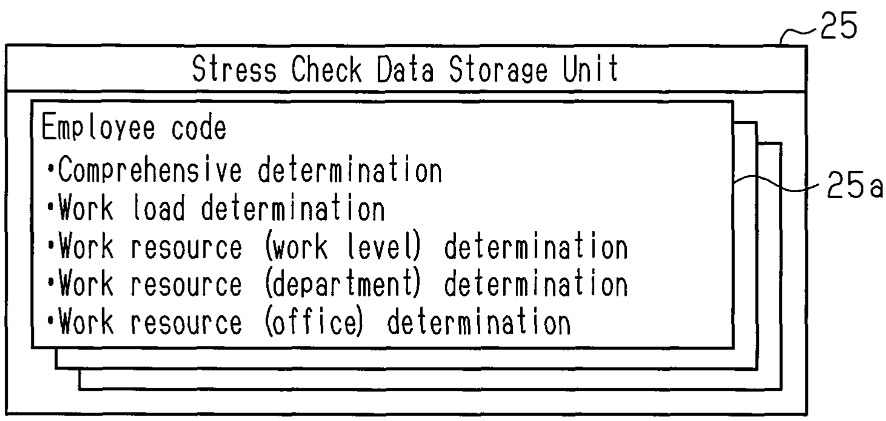


Fig.3C

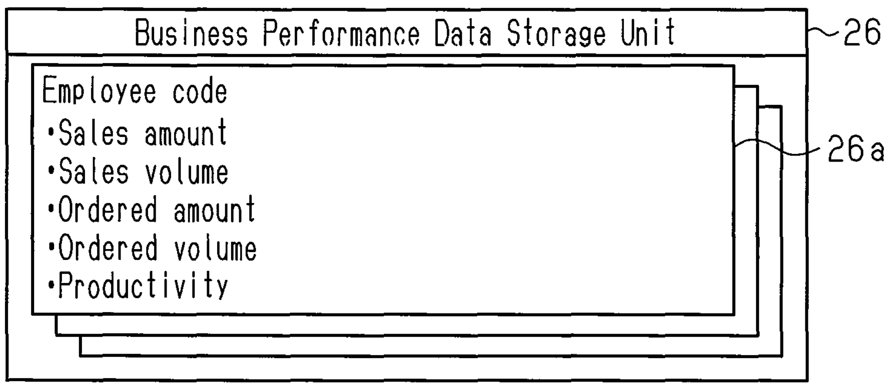

Fig.3D

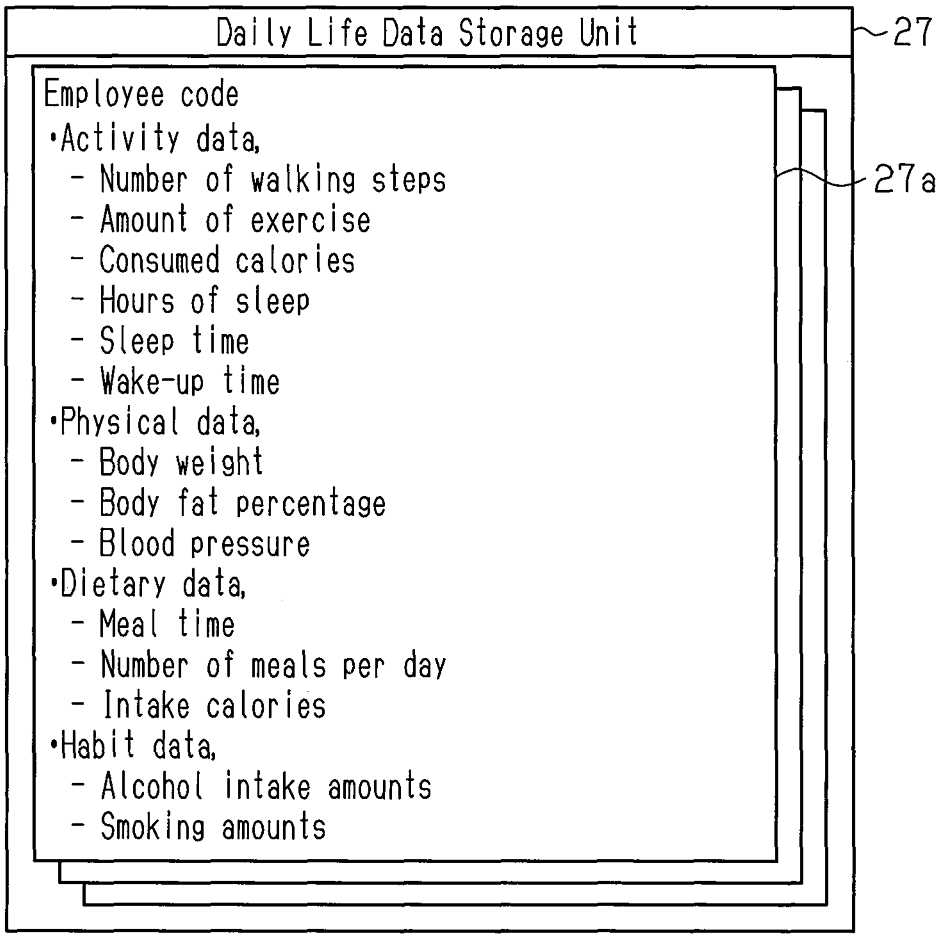
Daily Life Data Storage Unit
27
Employee code
•Activity data,
- Number of walking steps
- Amount of exercise
- Consumed calories
- Hours of sleep
- Sleep time
- Wake-up time
•Physical data,
- Body weight
- Body fat percentage
- Blood pressure
•Dietary data,
- Meal time
- Number of meals per day
- Intake calories
•Habit data,
- Alcohol intake amounts
- Smoking amounts
27a

Fig.3E

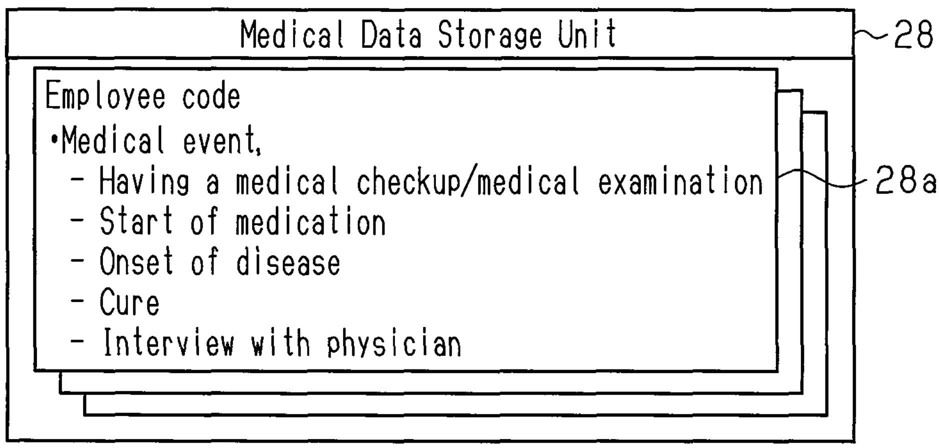
Medical Data Storage Unit
28
Employee code
•Medical event,
- Having a medical checkup/medical examination
- Start of medication
- Onset of disease
- Cure
- Interview with physician
28a

Fig.4

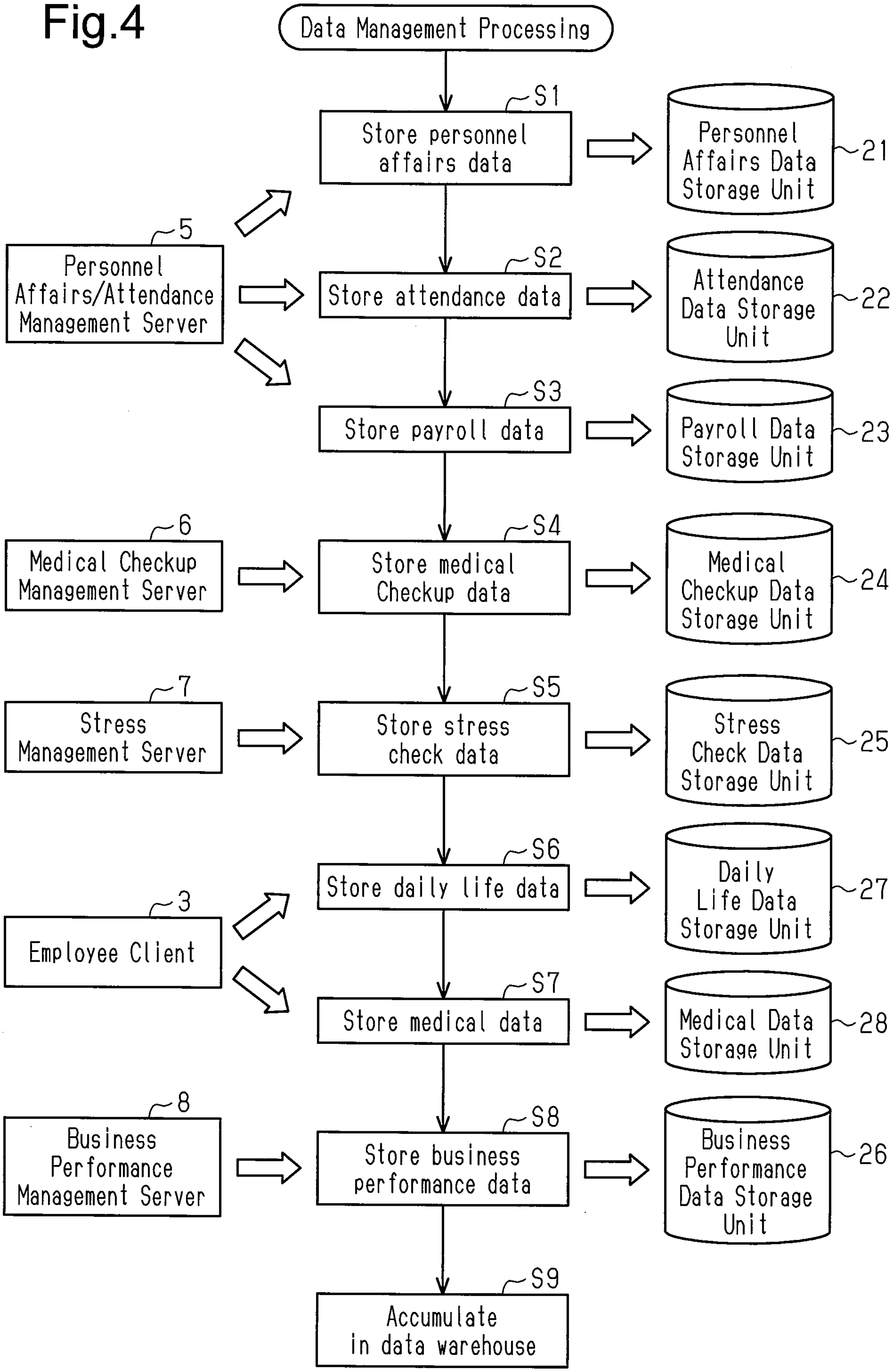
Data Management Processing
S1
Store personnel affairs data
Personnel Affairs Data Storage Unit
21
5
Personnel Affairs/Attendance Management Server
S2
Store attendance data
Attendance Data Storage Unit
22
S3
Store payroll data
Payroll Data Storage Unit
23
6
Medical Checkup Management Server
S4
Store medical Checkup data
Medical Checkup Data Storage Unit
24
7
Stress Management Server
S5
Store stress check data
Stress Check Data Storage Unit
25
3
Employee Client
S6
Store daily life data
Daily Life Data Storage Unit
27
S7
Store medical data
Medical Data Storage Unit
28
8
Business Performance Management Server
S8
Store business performance data
Business Performance Data Storage Unit
26
S9
Accumulate in data warehouse

Fig.5

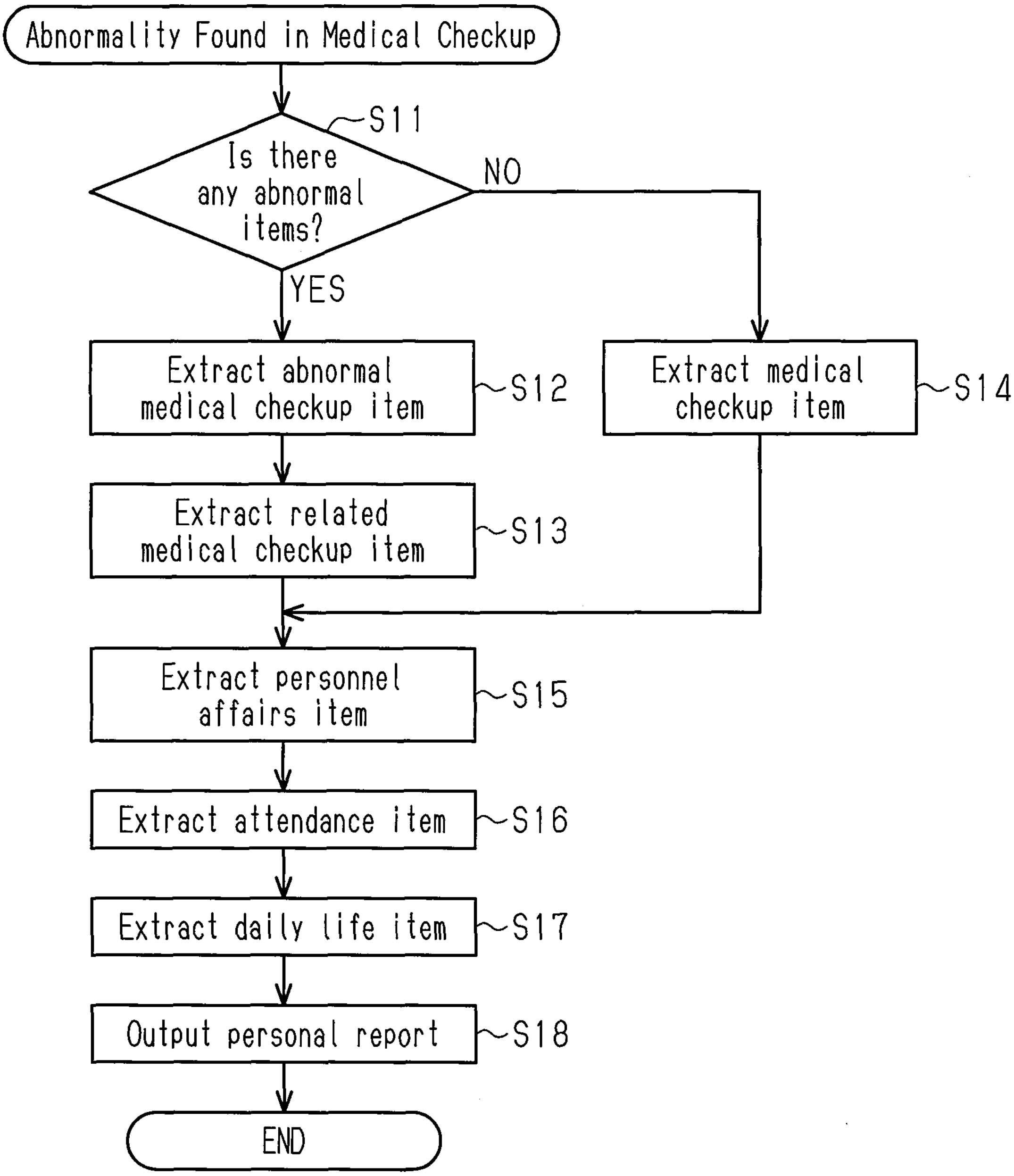
Abnormality Found in Medical Checkup
Is there any abnormal items?
S11
NO
YES
Extract abnormal medical checkup item
S12
Extract medical checkup item
S14
Extract related medical checkup item
S13
Extract personnel affairs item
S15
Extract attendance item
S16
Extract daily life item
S17
Output personal report
S18
END

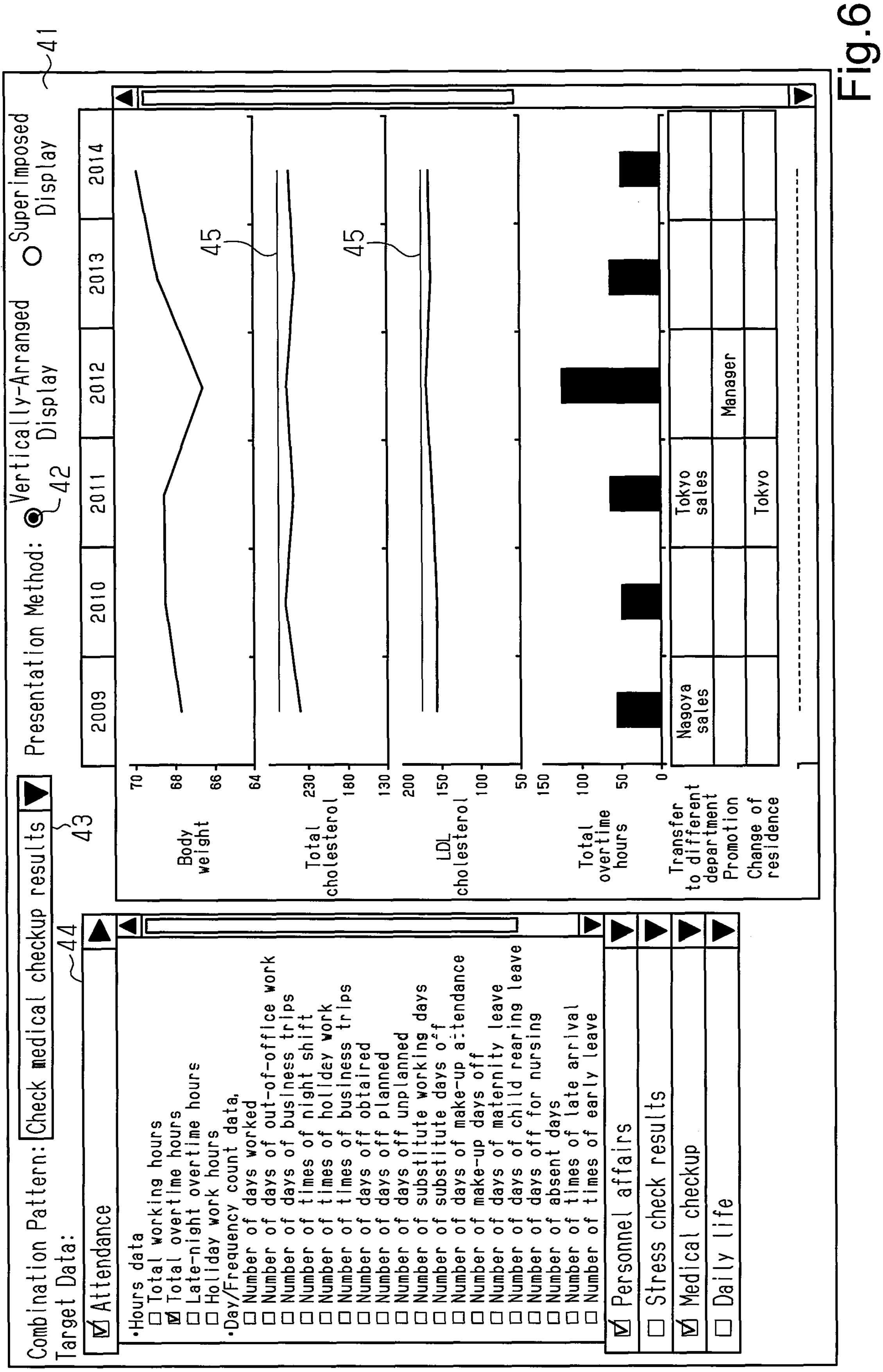
41
Combination Pattern: Check medical checkup results
43
44
Target Data:
Attendance
•Hours data
Total working hours
Total overtime hours
Late-night overtime hours
Holiday work hours
•Day/Frequency count data
Number of days worked
Number of days of out-of-office work
Number of days of business trips
Number of times of night shift
Number of times of holiday work
Number of times of business trips
Number of days off obtained
Number of days off planned
Number of days off unplanned
Number of substitute working days
Number of substitute days off
Number of days of make-up attendance
Number of make-up days off
Number of days of maternity leave
Number of days of child rearing leave
Number of days off for nursing
Number of absent days
Number of times of late arrival
Number of times of early leave
Personnel affairs
Stress check results
Medical checkup
Daily life
Presentation Method:
Vertically-Arranged Display
42
Superimposed Display
2009
2010
2011
2012
2013
2014
Body weight
70
68
66
64
Total cholesterol
230
180
130
LDL cholesterol
200
150
100
50
45
45
Total overtime hours
150
100
50
0
Transfer to different department
Promotion
Change of residence
Nagoya sales
Tokyo sales
Tokyo
Manager

Fig.6

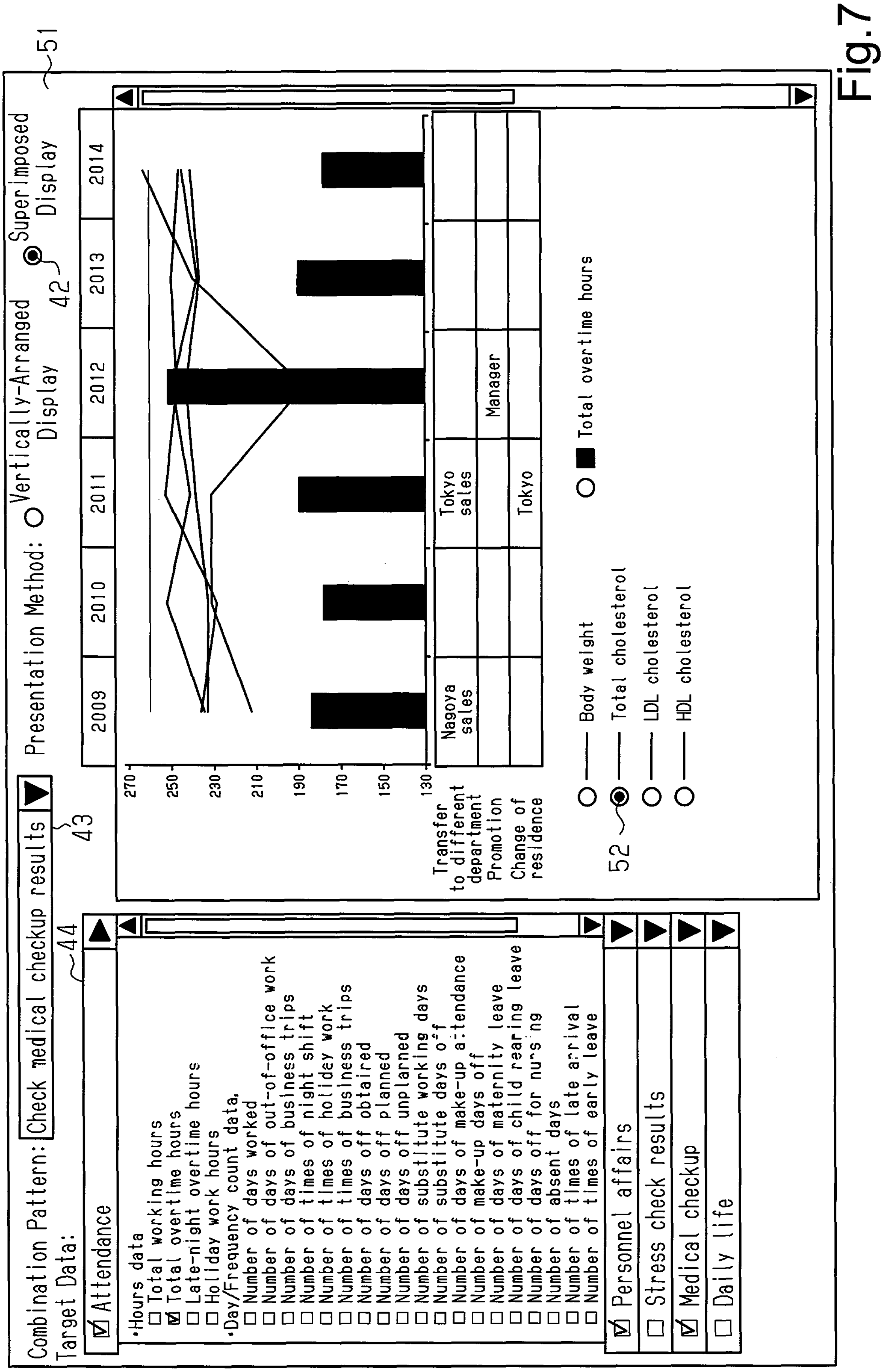

51
Combination Pattern: Check medical checkup results
43
Target Data:
44
Attendance
•Hours data
Total working hours
Total overtime hours
Late-night overtime hours
Holiday work hours
•Day/Frequency count data
Number of days worked
Number of days of out-of-office work
Number of days of business trips
Number of times of night shift
Number of times of holiday work
Number of times of business trips
Number of days off obtained
Number of days off planned
Number of days off unplanned
Number of substitute working days
Number of substitute days off
Number of days of make-up attendance
Number of make-up days off
Number of days of maternity leave
Number of days of child rearing leave
Number of days off for nursing
Number of absent days
Number of times of late arrival
Number of times of early leave
Personnel affairs
Stress check results
Medical checkup
Daily life
Presentation Method:
Vertically-Arranged Display
Superimposed Display
42
2009
2010
2011
2012
2013
2014
270
250
230
210
190
170
150
130
Transfer to different department
Promotion
Change of residence
Nagoya sales
Tokyo sales
Manager
Tokyo
Body weight
Total cholesterol
LDL cholesterol
HDL cholesterol
Total overtime hours
52


Fig.7

Fig.8

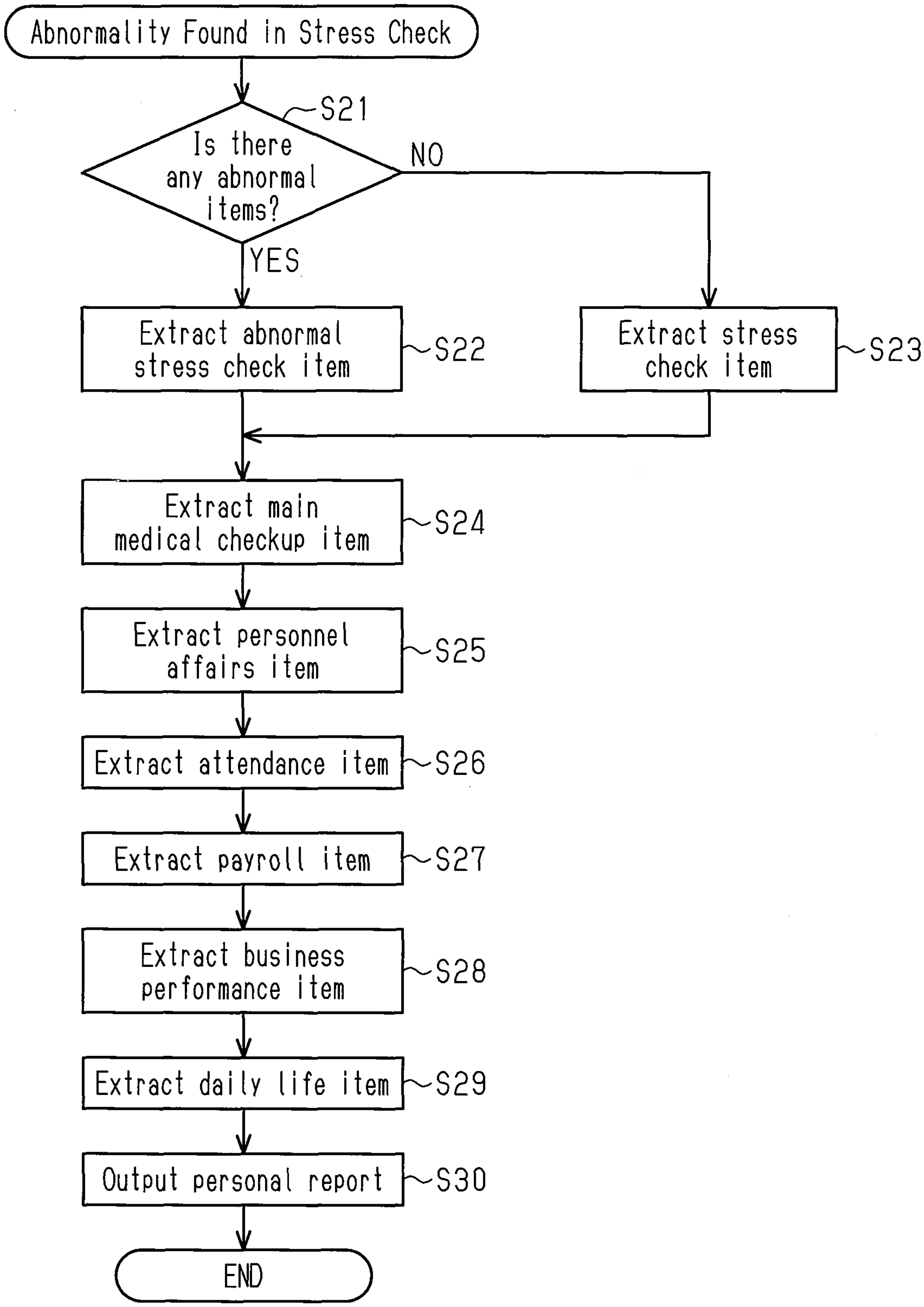
Abnormality Found in Stress Check
S21
Is there any abnormal items?
NO
YES
Extract abnormal stress check item
S22
Extract stress check item
S23
Extract main medical checkup item
S24
Extract personnel affairs item
S25
Extract attendance item
S26
Extract payroll item
S27
Extract business performance item
S28
Extract daily life item
S29
Output personal report
S30
END

Fig.9

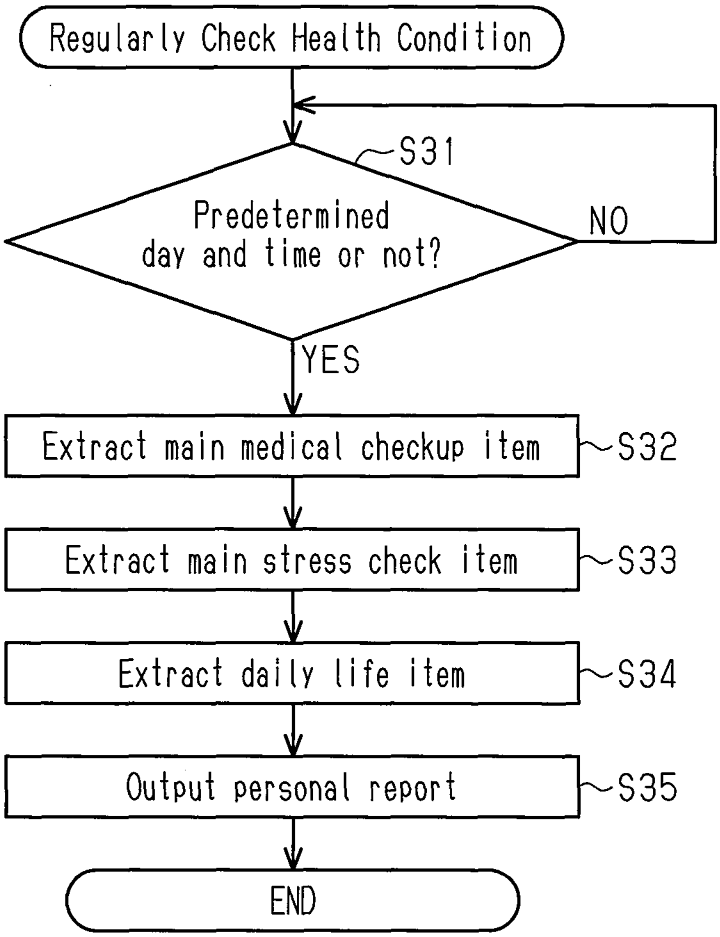
Regularly Check Health Condition
S31
Predetermined day and time or not?
NO
YES
Extract main medical checkup item
S32
Extract main stress check item
S33
Extract daily life item
S34
Output personal report
S35
END

Fig.10

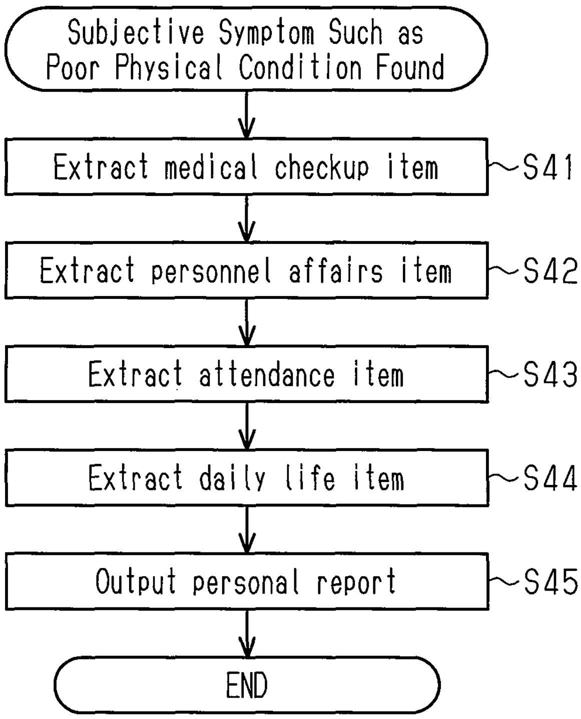
Subjective Symptom Such as Poor Physical Condition Found
Extract medical checkup item
S41
Extract personnel affairs item
S42
Extract attendance item
S43
Extract daily life item
S44
Output personal report
S45
END

Fig.11

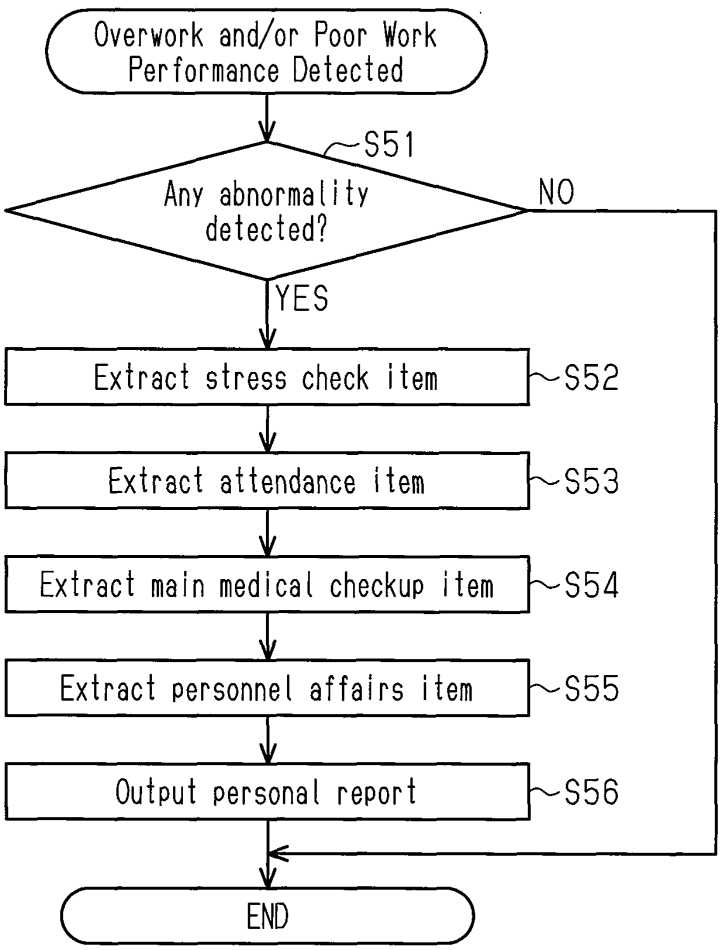
Overwork and/or Poor Work Performance Detected
S51
Any abnormality detected?
NO
YES
Extract stress check item
S52
Extract attendance item
S53
Extract main medical checkup item
S54
Extract personnel affairs item
S55
Output personal report
S56
END

Fig.12

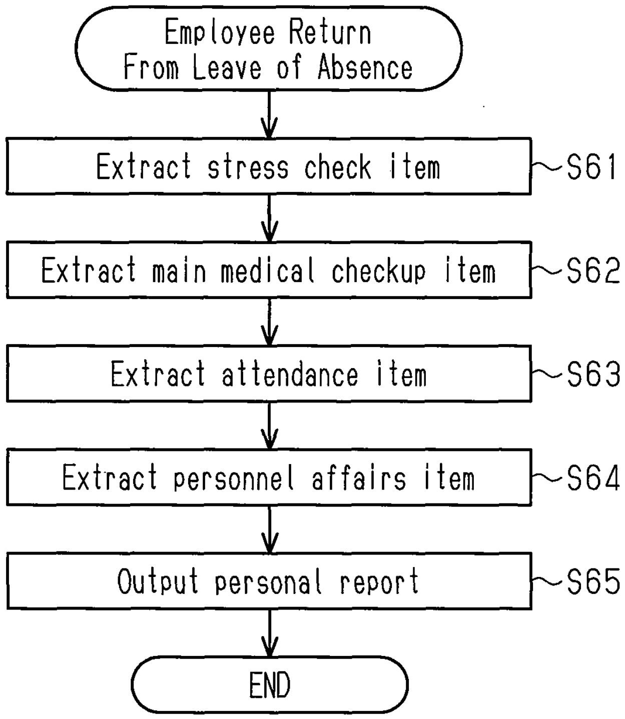
Employee Return From Leave of Absence
Extract stress check item
S61
Extract main medical checkup item
S62
Extract attendance item
S63
Extract personnel affairs item
S64
Output personal report
S65
END

Fig.13

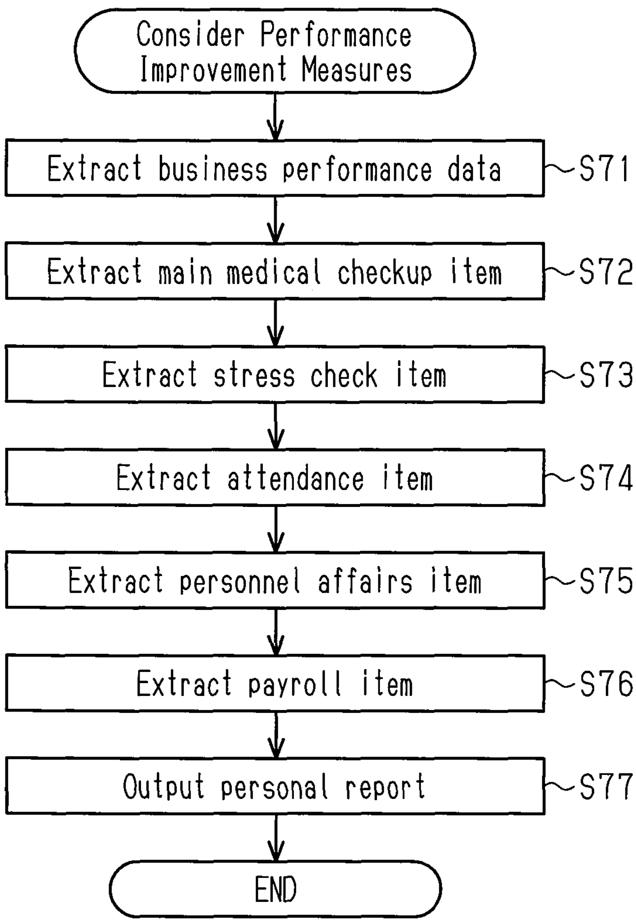
Consider Performance Improvement Measures
Extract business performance data
S71
Extract main medical checkup item
S72
Extract stress check item
S73
Extract attendance item
S74
Extract personnel affairs item
S75
Extract payroll item
S76
Output personal report
S77
END

Fig.14

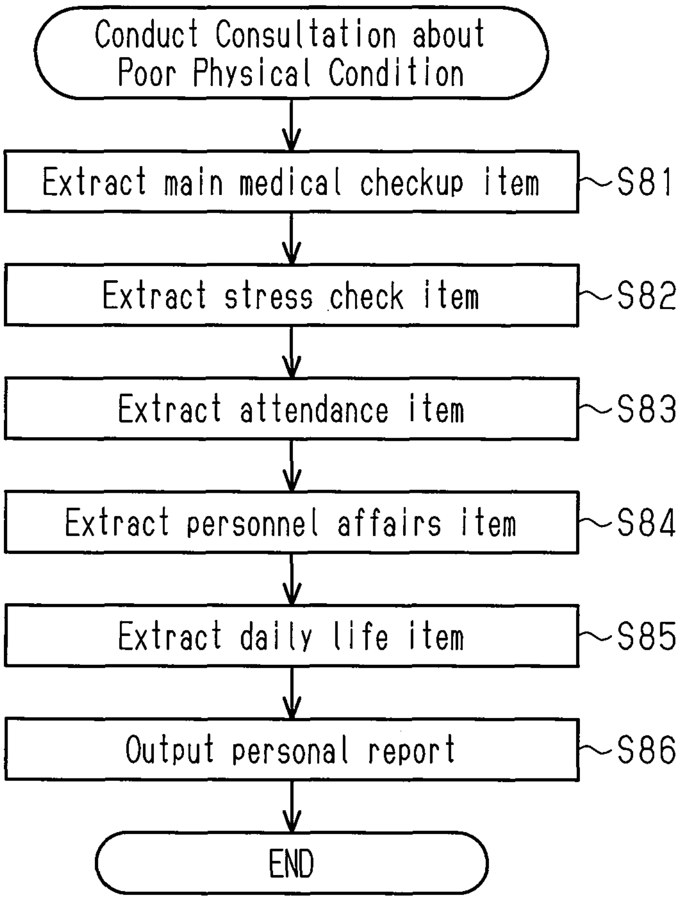
Conduct Consultation about Poor Physical Condition
Extract main medical checkup item
S81
Extract stress check item
S82
Extract attendance item
S83
Extract personnel affairs item
S84
Extract daily life item
S85
Output personal report
S86
END

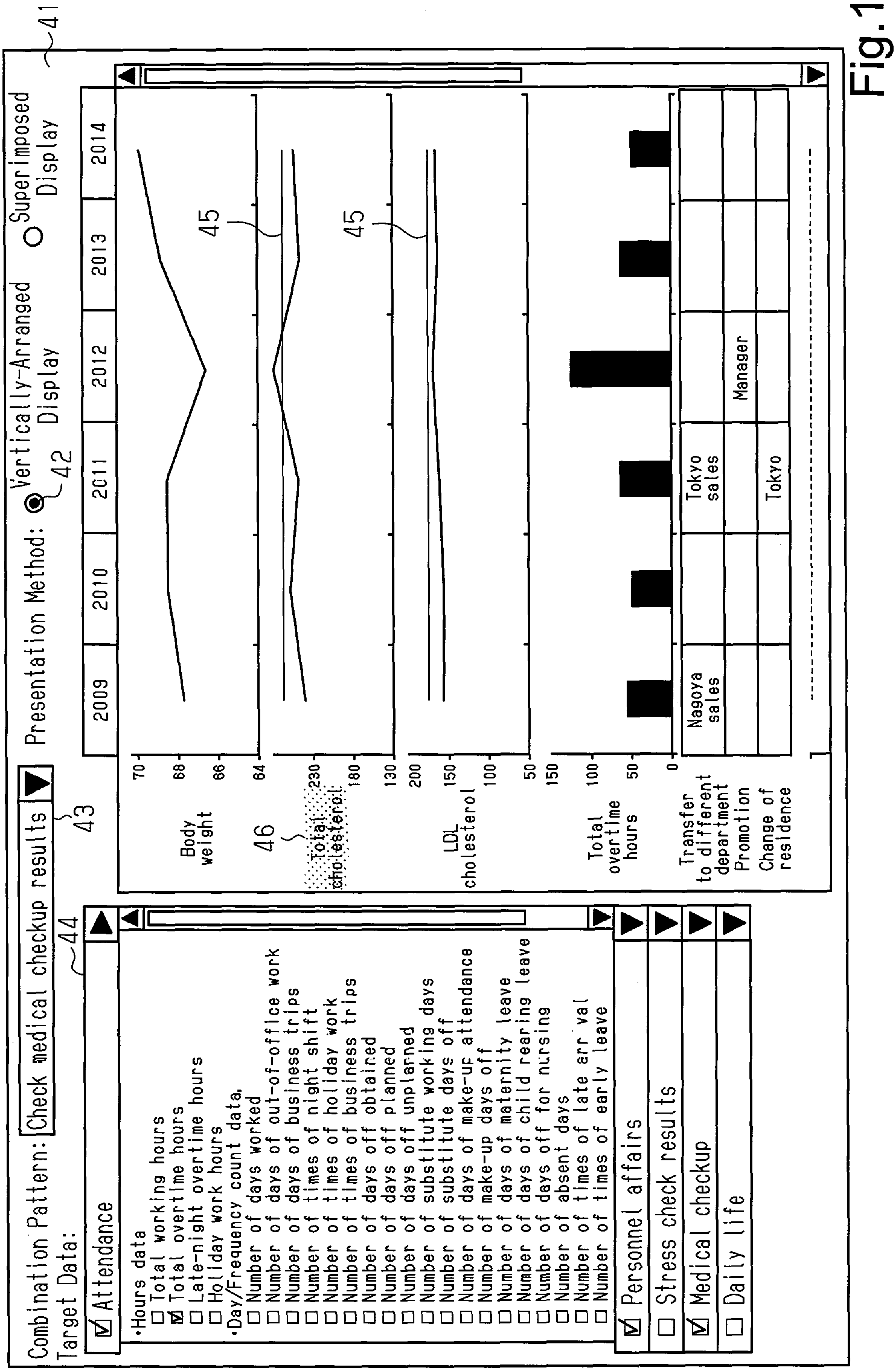
Combination Pattern:
Check medical checkup results
Target Data:
Presentation Method:
Vertically-Arranged Display
Superimposed Display
Attendance
•Hours data
Total working hours
Total overtime hours
Late-night overtime hours
Holiday work hours
•Day/Frequency count data
Number of days worked
Number of days of out-of-office work
Number of days of business trips
Number of times of night shift
Number of times of holiday work
Number of times of business trips
Number of days off obtained
Number of days off planned
Number of days off unplanned
Number of substitute working days
Number of substitute days off
Number of days of make-up attendance
Number of make-up days off
Number of days of maternity leave
Number of days of child rearing leave
Number of days off for nursing
Number of absent days
Number of times of late arrival
Number of times of early leave
Personnel affairs
Stress check results
Medical checkup
Daily life
2009
2010
2011
2012
2013
2014
Body weight
70
68
66
64
Total cholesterol
230
180
130
LDL cholesterol
200
150
100
50
Total overtime hours
150
100
50
0
Transfer to different department
Promotion
Change of residence
Nagoya sales
Tokyo sales
Manager
Tokyo
41
42
43
44
45
45
46

Fig.15

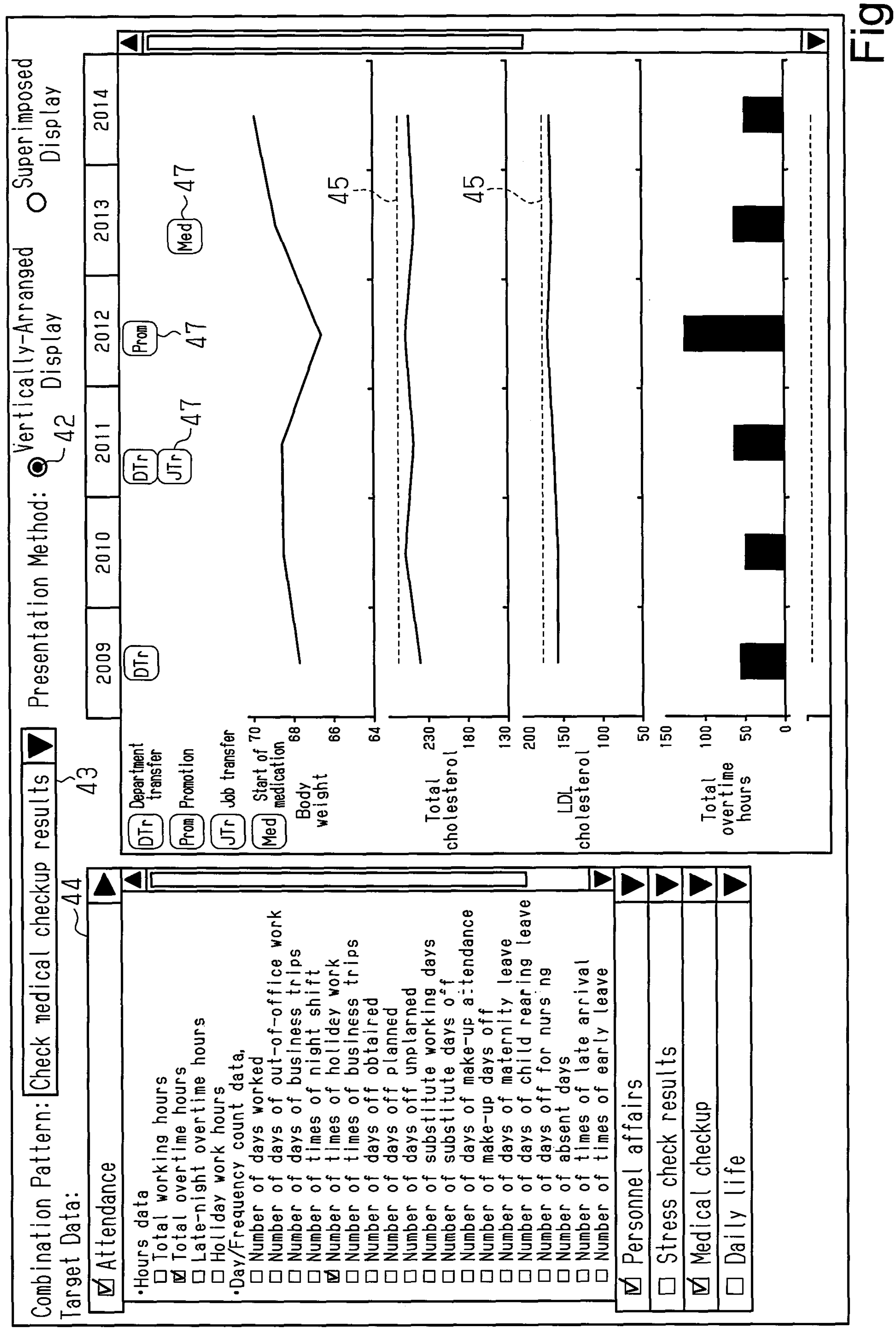
Combination Pattern: Check medical checkup results
Presentation Method: Vertically-Arranged Display
Superimposed Display
42
43
44
45
47
Target Data:
Attendance
•Hours data
Total working hours
Total overtime hours
Late-night overtime hours
Holiday work hours
•Day/Frequency count data,
Number of days worked
Number of days of out-of-office work
Number of days of business trips
Number of times of night shift
Number of times of holiday work
Number of times of business trips
Number of days off obtained
Number of days off planned
Number of days off unplanned
Number of substitute working days
Number of substitute days off
Number of days of make-up attendance
Number of make-up days off
Number of days of maternity leave
Number of days of child rearing leave
Number of days off for nursing
Number of absent days
Number of times of late arrival
Number of times of early leave
Personnel affairs
Stress check results
Medical checkup
Daily life
2009
2010
2011
2012
2013
2014
DTr Department transfer
Prom Promotion
JTr Job transfer
Med Start of medication
DTr
JTr
Prom
Med
Body weight
70
68
66
64
Total cholesterol
230
180
130
LDL cholesterol
200
150
100
50
Total overtime hours
150
100
50
0

Fig.16

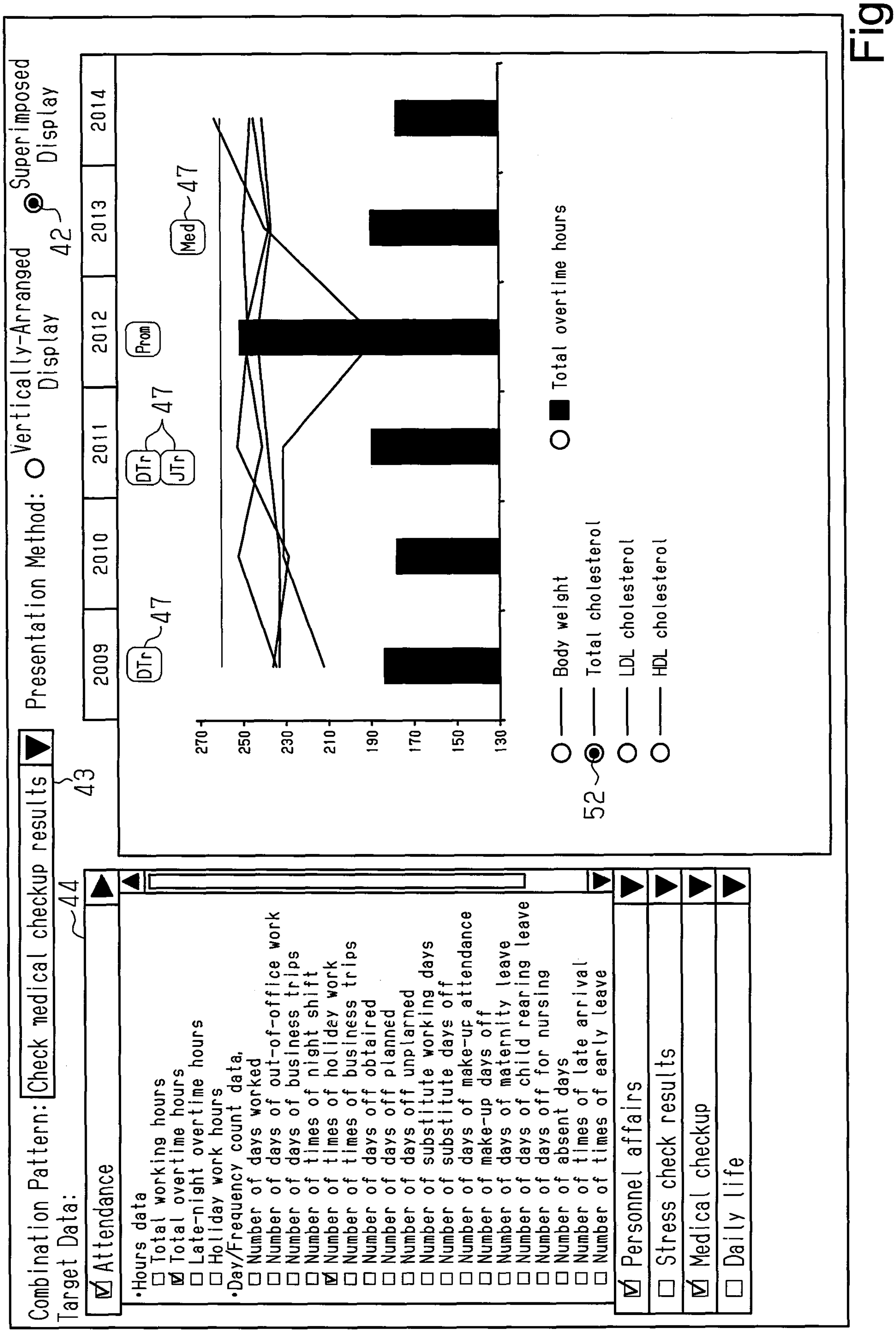
Combination Pattern: Check medical checkup results
43
Target Data:
44
Attendance
•Hours data
Total working hours
Total overtime hours
Late-night overtime hours
Holiday work hours
•Day/Frequency count data
Number of days worked
Number of days of out-of-office work
Number of days of business trips
Number of times of night shift
Number of times of holiday work
Number of times of business trips
Number of days off obtained
Number of days off planned
Number of days off unplanned
Number of days of substitute working days
Number of substitute days off
Number of days of make-up attendance
Number of make-up days off
Number of days of maternity leave
Number of days of child rearing leave
Number of days off for nursing
Number of absent days
Number of times of late arrival
Number of times of early leave
Personnel affairs
Stress check results
Medical checkup
Daily life
Presentation Method:
Vertically-Arranged Display
42
Superimposed Display
2009
2010
2011
2012
2013
2014
DTr
47
DTr
JTr
47
Prom
Med
47
270
250
230
210
190
170
150
130
Body weight
Total cholesterol
LDL cholesterol
HDL cholesterol
52
Total overtime hours

Fig.17

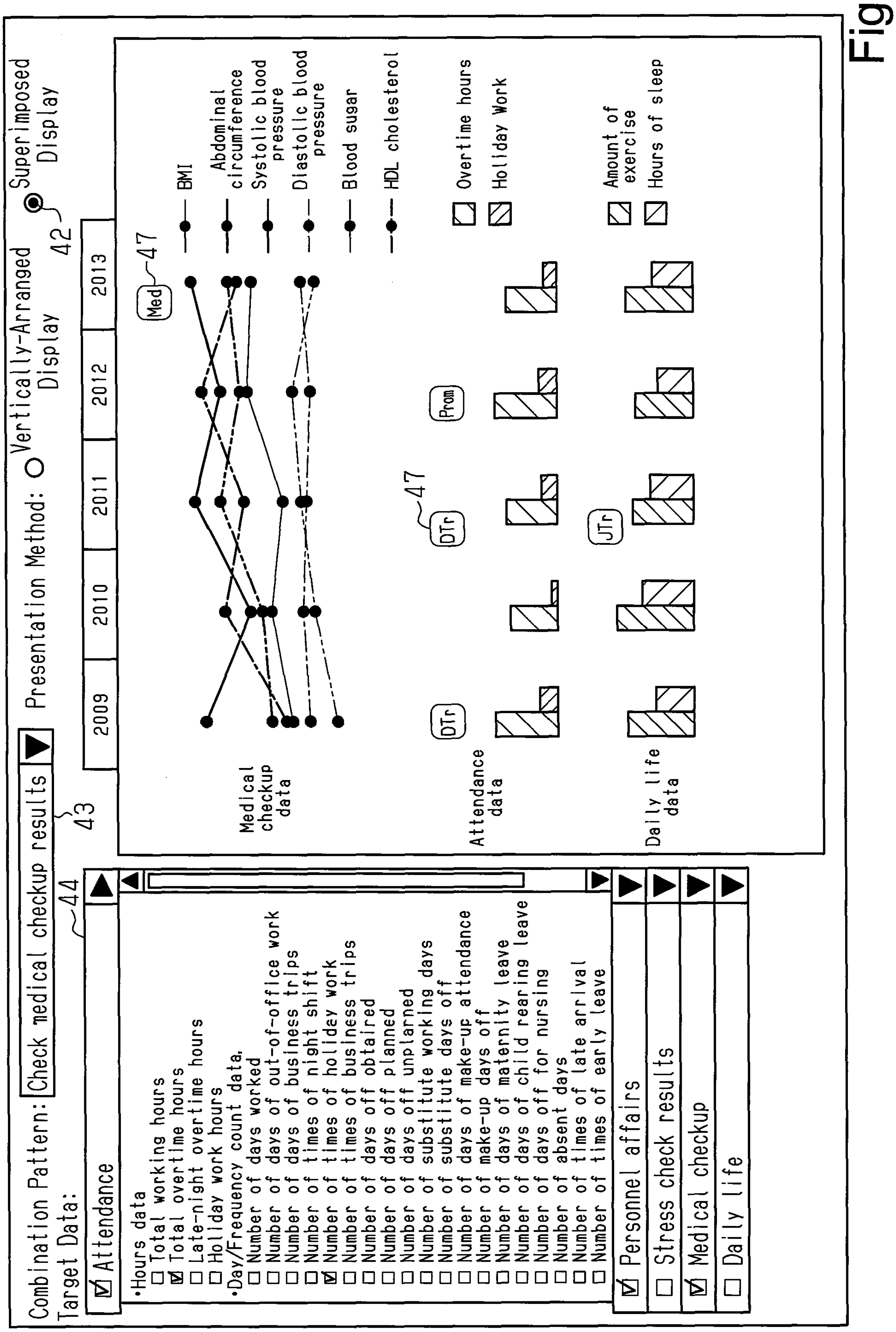
Combination Pattern: Check medical checkup results
Presentation Method: Vertically-Arranged Display
Superimposed Display
Target Data:
Attendance
•Hours data
Total working hours
Total overtime hours
Late-night overtime hours
Holiday work hours
•Day/Frequency count data
Number of days worked
Number of days of out-of-office work
Number of days of business trips
Number of times of night shift
Number of times of holiday work
Number of times of business trips
Number of days off obtained
Number of days off planned
Number of days off unplanned
Number of substitute working days
Number of substitute days off
Number of days of make-up attendance
Number of make-up days off
Number of days of maternity leave
Number of days of child rearing leave
Number of days off for nursing
Number of absent days
Number of times of late arrival
Number of times of early leave
Personnel affairs
Stress check results
Medical checkup
Daily life
2009
2010
2011
2012
2013
Medical checkup data
Attendance data
Daily life data
DTr
JTr
Prom
Med
BMI
Abdominal circumference
Systolic blood pressure
Diastolic blood pressure
Blood sugar
HDL cholesterol
Overtime hours
Holiday Work
Amount of exercise
Hours of sleep
42
43
44
47

Fig.18

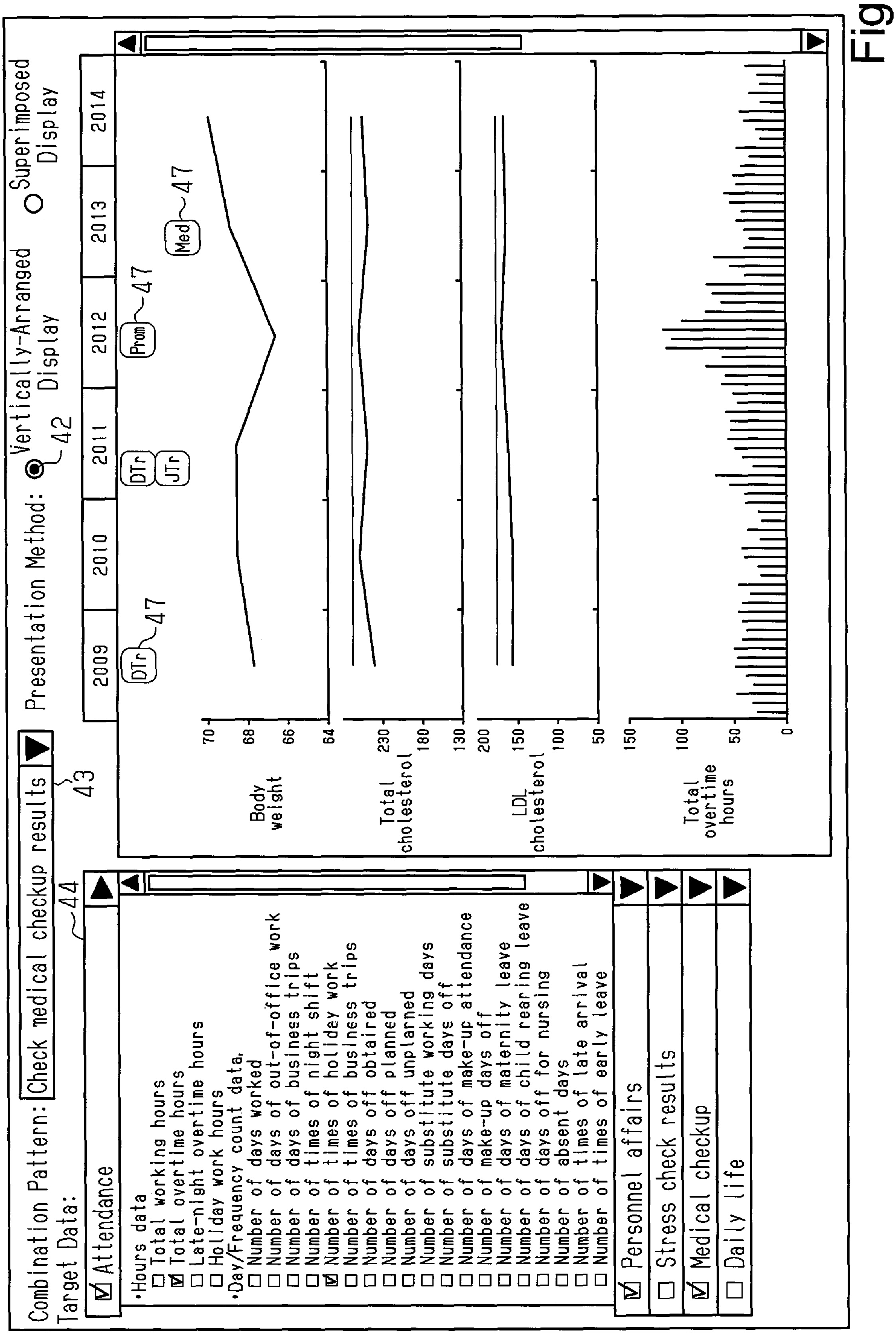

Combination Pattern: Check medical checkup results
43
Target Data:
44
Attendance
•Hours data
Total working hours
Total overtime hours
Late-night overtime hours
Holiday work hours
•Day/Frequency count data,
Number of days worked
Number of days of out-of-office work
Number of days of business trips
Number of times of night shift
Number of times of holiday work
Number of times of business trips
Number of days off obtained
Number of days off planned
Number of days off unplanned
Number of substitute working days
Number of substitute days off
Number of days of make-up attendance
Number of make-up days off
Number of days of maternity leave
Number of days of child rearing leave
Number of days off for nursing
Number of absent days
Number of times of late arrival
Number of times of early leave
Personnel affairs
Stress check results
Medical checkup
Daily life
Presentation Method:
Vertically-Arranged Display
42
Superimposed Display
2009
2010
2011
2012
2013
2014
DTr
47
DTr
JTr
Prom
47
Med
47
Body weight
70
68
66
64
Total cholesterol
230
180
130
LDL cholesterol
200
150
100
50
Total overtime hours
150
100
50
0


Fig.19

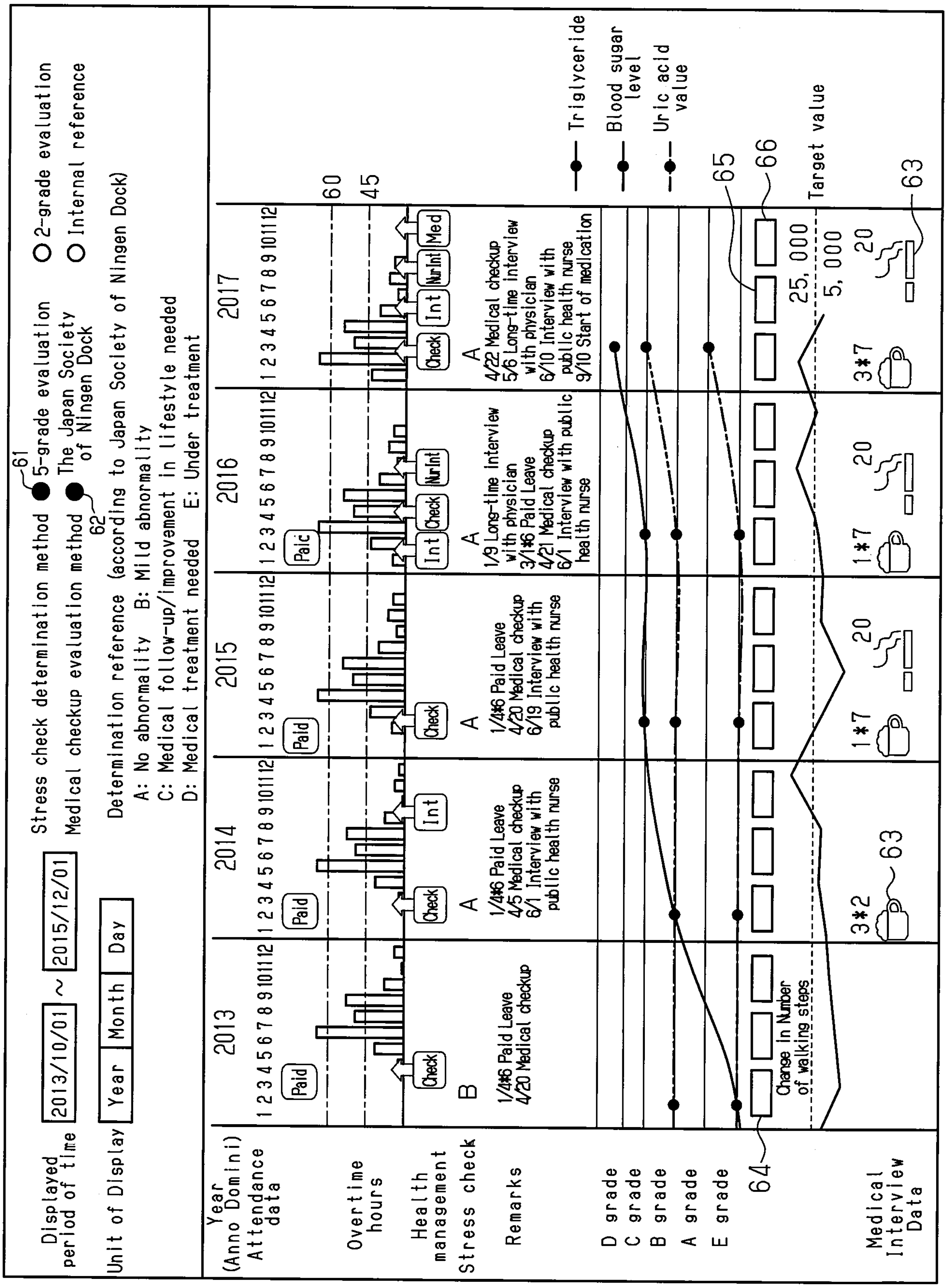

Displayed period of time
2013/10/01 ~ 2015/12/01
Unit of Display
Year
Month
Day
Stress check determination method
5-grade evaluation
2-grade evaluation
61
Medical checkup evaluation method
The Japan Society of Ningen Dock
Internal reference
62
Determination reference (according to Japan Society of Ningen Dock)
A: No abnormality B: Mild abnormality
C: Medical follow-up/improvement in lifestyle needed
D: Medical treatment needed E: Under treatment
Year (Anno Domini)
Attendance data
Overtime hours
Health management
Stress check
Remarks
D grade
C grade
B grade
A grade
E grade
64
Medical Interview Data
2013
2014
2015
2016
2017
1 2 3 4 5 6 7 8 9 10 11 12
60
45
Paid
Check
Int
NurInt
Med
B
A
1/4*6 Paid Leave
4/20 Medical checkup
1/4*6 Paid Leave
4/5 Medical checkup
6/1 Interview with public health nurse
1/4*6 Paid Leave
4/20 Medical checkup
6/19 Interview with public health nurse
1/9 Long-time interview with physician
3/1*6 Paid Leave
4/21 Medical checkup
6/1 Interview with public health nurse
4/22 Medical checkup
5/6 Long-time interview with physician
6/10 Interview with public health nurse
9/10 Start of medication
Triglyceride
Blood sugar level
Uric acid value
65
66
Target value
Change in Number of walking steps
25, 000
5, 000
3*2
1*7
3*7
20
63


Fig.20A

Fig.20B

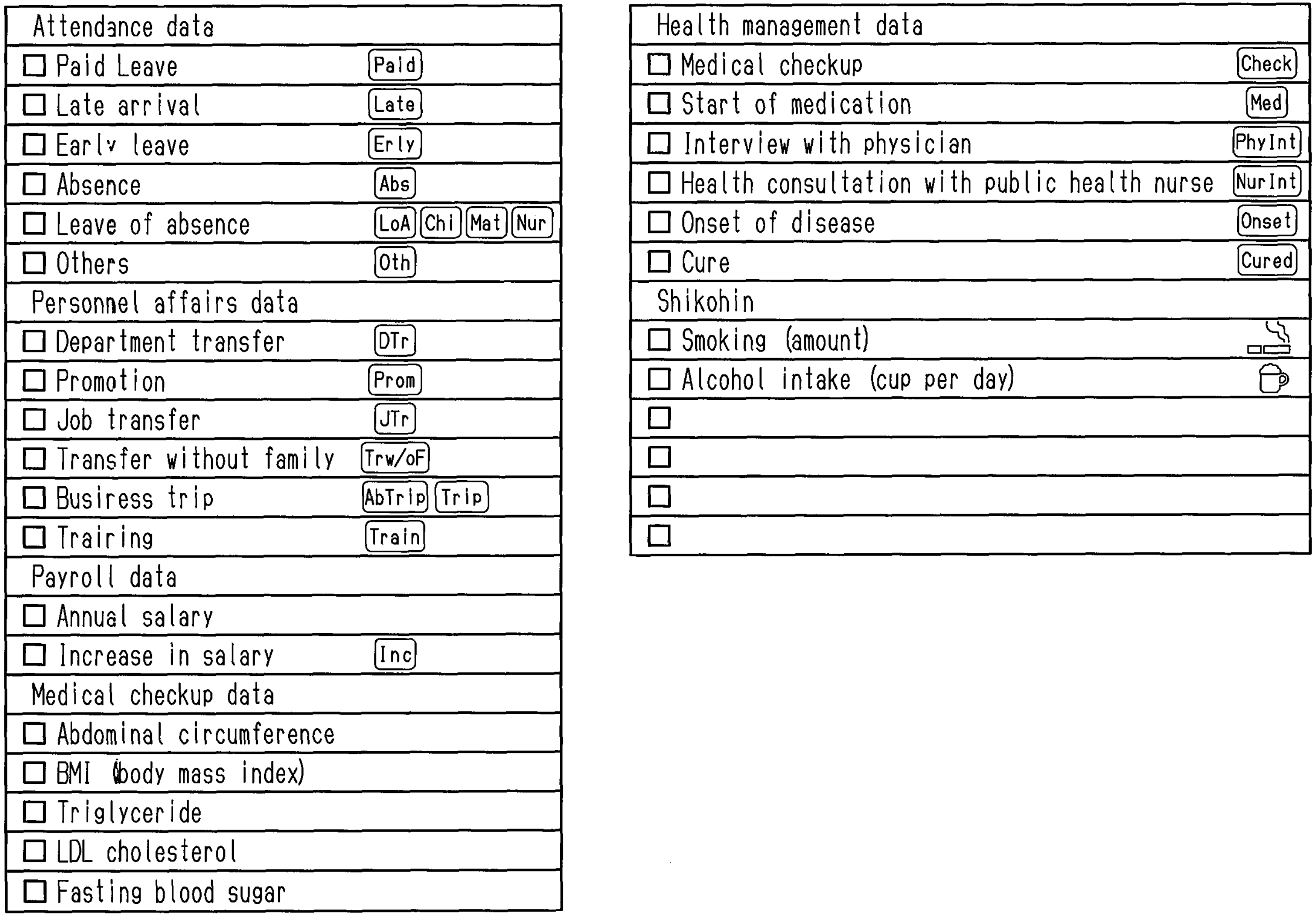
Attendance data
Paid Leave
Paid
Late arrival
Late
Early leave
Erly
Absence
Abs
Leave of absence
LoA
Chi
Mat
Nur
Others
Oth
Personnel affairs data
Department transfer
DTr
Promotion
Prom
Job transfer
JTr
Transfer without family
Trw/oF
Business trip
AbTrip
Trip
Training
Train
Payroll data
Annual salary
Increase in salary
Inc
Medical checkup data
Abdominal circumference
BMI (body mass index)
Triglyceride
LDL cholesterol
Fasting blood sugar
Health management data
Medical checkup
Check
Start of medication
Med
Interview with physician
PhyInt
Health consultation with public health nurse
NurInt
Onset of disease
Onset
Cure
Cured
Shikohin
Smoking (amount)
Alcohol intake (cup per day)

Fig.21

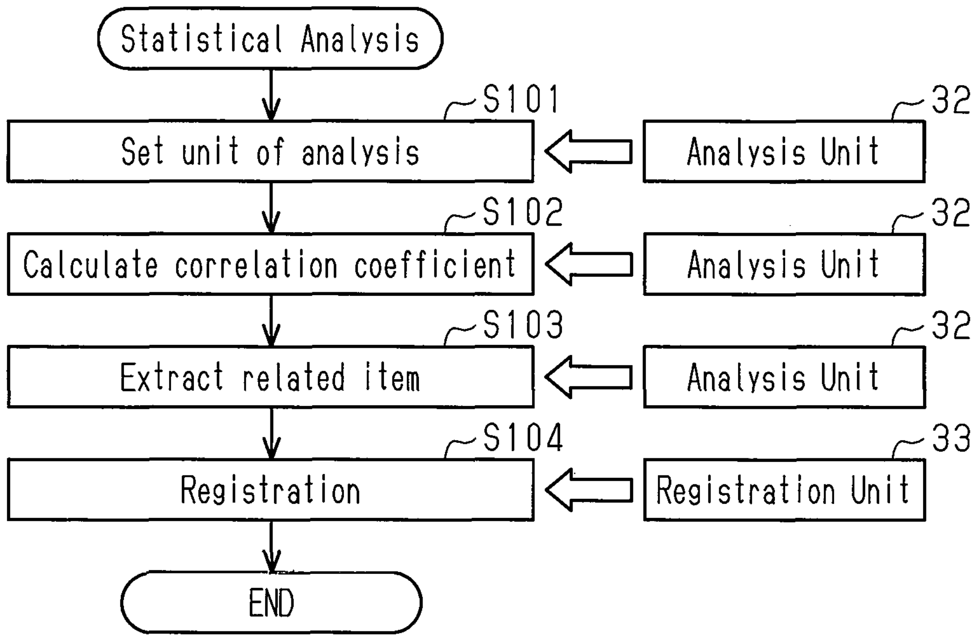
Statistical Analysis
S101
Set unit of analysis
32
Analysis Unit
S102
Calculate correlation coefficient
32
Analysis Unit
S103
Extract related item
32
Analysis Unit
S104
Registration
33
Registration Unit
END

Fig.22

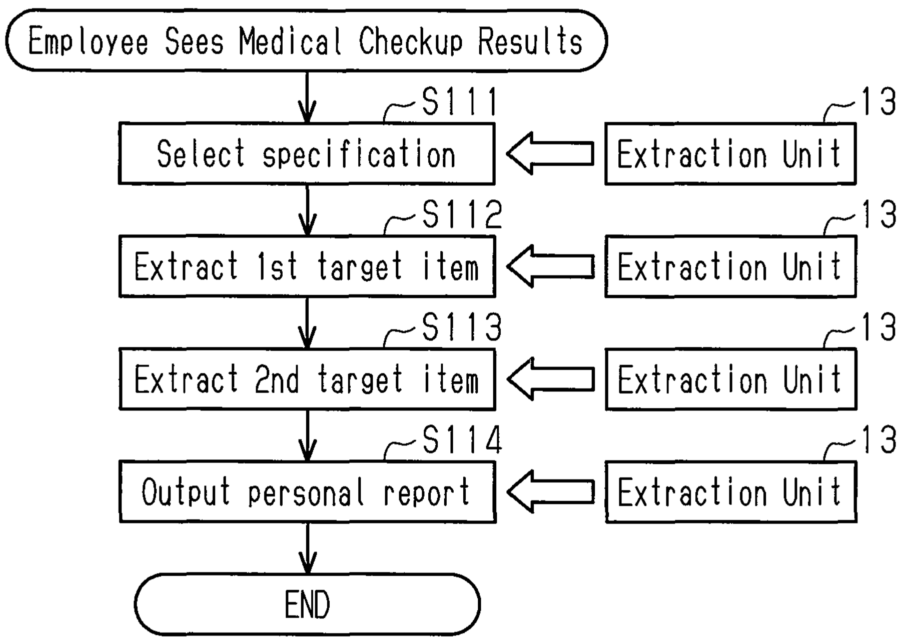
Employee Sees Medical Checkup Results
S111
Select specification
13
Extraction Unit
S112
Extract 1st target item
13
Extraction Unit
S113
Extract 2nd target item
13
Extraction Unit
S114
Output personal report
13
Extraction Unit
END

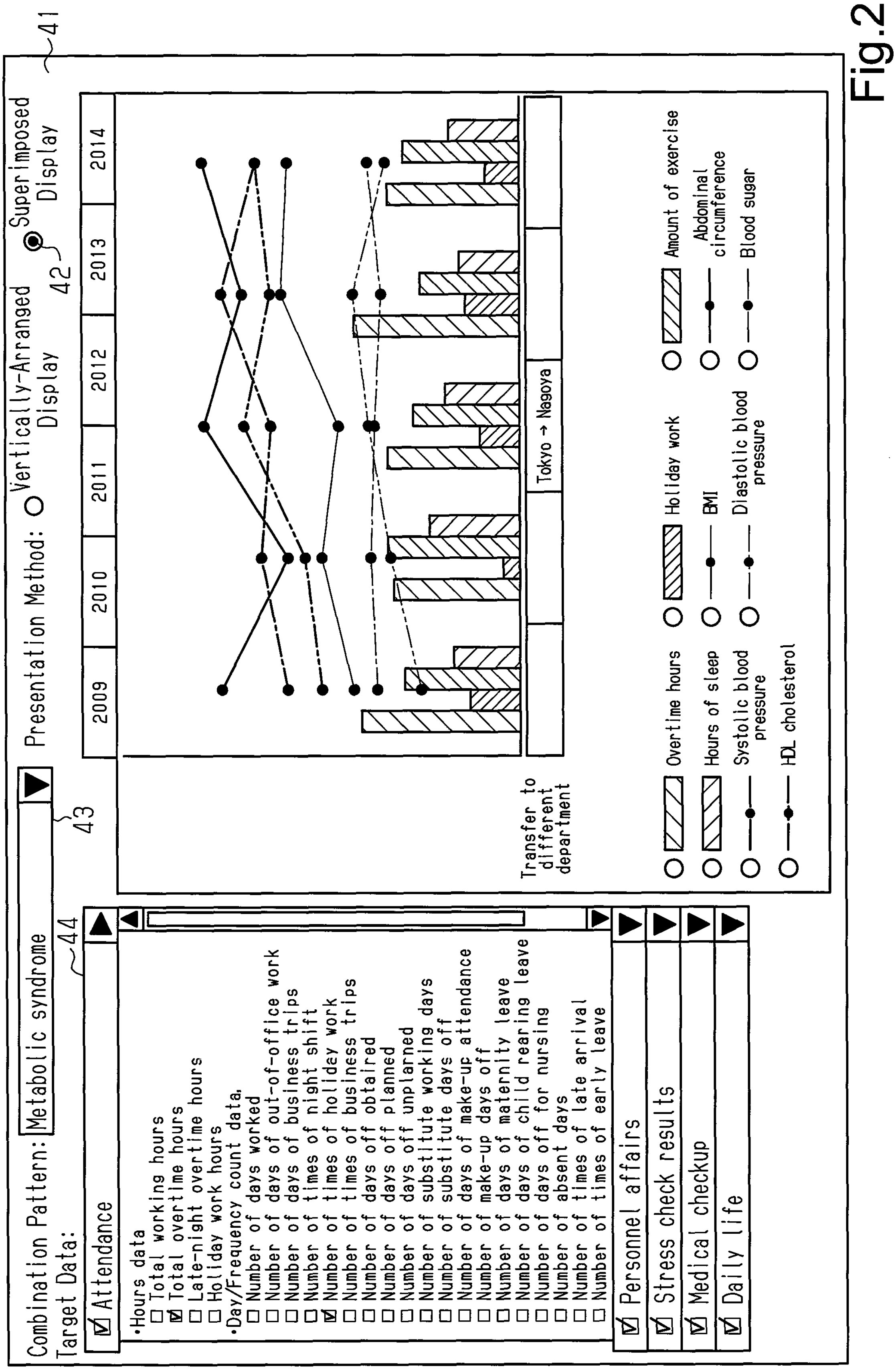
41
Combination Pattern: Metabolic syndrome
43
Presentation Method: Vertically-Arranged Display
Superimposed Display
42
Target Data:
44
Attendance
•Hours data
Total working hours
Total overtime hours
Late-night overtime hours
Holiday work hours
•Day/Frequency count data.
Number of days worked
Number of days of out-of-office work
Number of days of business trips
Number of times of night shift
Number of times of holiday work
Number of times of business trips
Number of days off obtained
Number of days off planned
Number of days off unplanned
Number of substitute working days
Number of substitute days off
Number of days of make-up attendance
Number of make-up days off
Number of days of maternity leave
Number of days of child rearing leave
Number of days off for nursing
Number of absent days
Number of times of late arrival
Number of times of early leave
Personnel affairs
Stress check results
Medical checkup
Daily life
2009
2010
2011
2012
2013
2014
Transfer to different department
Tokyo → Nagoya
Overtime hours
Holiday work
Amount of exercise
Hours of sleep
BMI
Abdominal circumference
Systolic blood pressure
Diastolic blood pressure
Blood sugar
HDL cholesterol

Fig.23

Fig.24

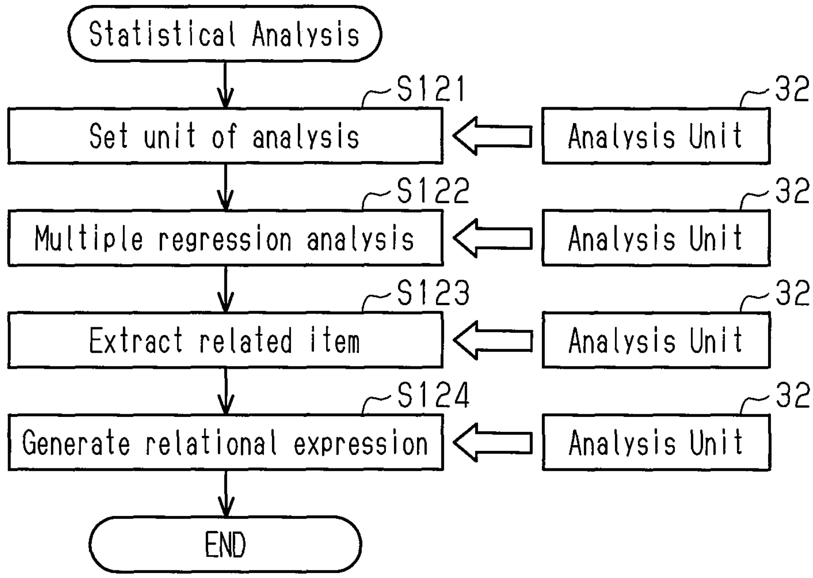


Fig.25

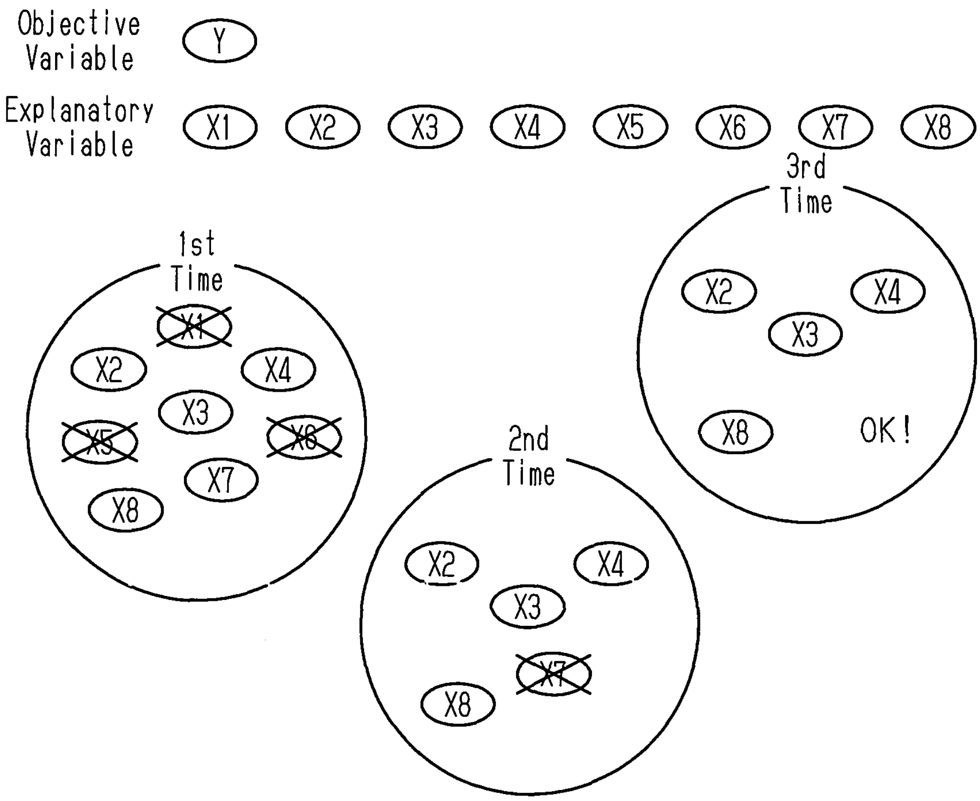


Relational Expression Y=aX2+bX3+cX4+dX8+e

where a, b, c, and d are slopes, and e is an intercept

Fig.26

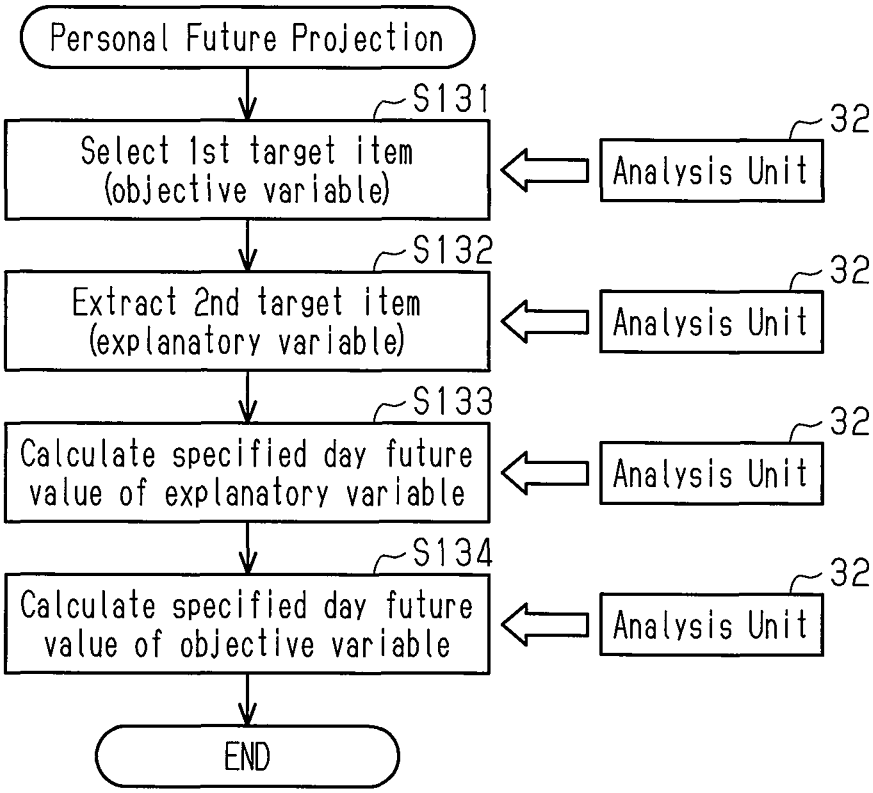
Personal Future Projection
S131
Select 1st target item (objective variable)
32
Analysis Unit
S132
Extract 2nd target item (explanatory variable)
32
Analysis Unit
S133
Calculate specified day future value of explanatory variable
32
Analysis Unit
S134
Calculate specified day future value of objective variable
32
Analysis Unit
END

## INTERNATIONAL SEARCH REPORT

International application No. PCT/JP2016/079152

**A. CLASSIFICATION OF SUBJECT MATTER**

*G06Q10/10*(2012.01)i, *A61B5/00*(2006.01)i

According to International Patent Classification (IPC) or to both national classification and IPC

**B. FIELDS SEARCHED**

Minimum documentation searched (classification system followed by classification symbols)

G06Q10/00-99/00, A61B5/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2015/111548 A1 (Nittsu System Co., Ltd.), 30 July 2015 (30.07.2015), paragraphs [0009] to [0014], [0023] to [0105] & JP 2015-158895 A | 1-16 |
| Y | Minako TOBA et al., "Correlation Analysis between Office Workers' Stress and Features Extracted from PC-Operation Logs", The Transactions of the Institute of Electronics, Information and Communication Engineers, 01 April 2012 (01.04.2012), vol.J95-D, no.4, pages 747 to 757, ISSN 1880-4535 | 1-16 |

☒ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 16 November 2016 (16.11.16) | 29 November 2016 (29.11.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No.
PCT/JP2016/079152

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2002-24407 A (Misawa Ban Kabushiki Kaisha), 25 January 2002 (25.01.2002), paragraphs [0027] to [0034], [0042] to [0046], [0056] to [0057]; fig. 16 (Family: none) | 1-16 |
| Y | JP 2011-150395 A (Toshiba Sumiden Medical Information Systems Corp.), 04 August 2011 (04.08.2011), paragraphs [0023] to [0040]; fig. 5, 7 to 9 (Family: none) | 5-6 |
| Y | JP 2006-293766 A (Aruze Corp.), 26 October 2006 (26.10.2006), paragraphs [0055] to [0056], [0065] to [0066]; fig. 12 (Family: none) | 6,9-16 |
| Y | JP 2007-188365 A (Takasaki University of Health and Welfare), 26 July 2007 (26.07.2007), paragraphs [0009] to [0015] (Family: none) | 6,9-16 |
| Y | JP 2006-255028 A (Nippon Telegraph and Telephone Corp.), 28 September 2006 (28.09.2006), paragraphs [0063] to [0073] (Family: none) | 12-14 |
| Y | Katsuhiko TSUJINO et al., "Health Promotion System", 2005 Nendo Proceedings of the Annual Conference of JSAI (Dai 19 Kai), 15 June 2005 (15.06.2005), 2G1-04, pages 1 to 4 | 12-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003256578 A